# EUROPEAN PATENT SPECIFICATION

(11) **EP 4 108 279 B1**
(45) Date of publication and mention of the grant of the patent: **09.04.2025**
(21) Application number: 20920396.7
(22) Date of filing: 24.08.2020
(51) Int. Cl.: A61M 11/00, A61M 15/00, A24D 3/18, A24F 40/485, A61M 15/06

(54) **ELECTRONIC ATOMIZATION DEVICE AND AIR CURTAIN FORMATION STRUCTURE USED BY SAME**
ELEKTRONISCHE ZERSTÄUBUNGSVORRICHTUNG UND LUFTVORHANGBILDUNGSSTRUKTUR DAMIT
DISPOSITIF D'ATOMISATION ÉLECTRONIQUE ET STRUCTURE DE FORMATION DE RIDEAU D'AIR UTILISÉE PAR CELUI-CI

(30) Priority: 20.02.2020 CN 202010105137
(43) Date of publication of application: 28.12.2022
(73) Proprietor: Shenzhen Smoore Technology Limited, Shenzhen, Guangdong 518102 (CN)
(72) Inventor: LEI, Guilin, Shenzhen, Guangdong 518102 (CN); XU, Dan, Shenzhen, Guangdong 518102 (CN); TANG, Guangwu, Shenzhen, Guangdong 518102 (CN); JIANG, Ru, Shenzhen, Guangdong 518102 (CN); GONG, Boxue, Shenzhen, Guangdong 518102 (CN); CHENG, Shiyi, Shenzhen, Guangdong 518102 (CN); XIE, Pan, Shenzhen, Guangdong 518102 (CN)
(74) Representative: Herrero & Asociados, S.L.
(86) International application number: PCT/CN2020/110870
(87) International publication number: WO 2021/164223

(56) References cited:
- WO-A1-2014/029827
- WO-A1-2015/112750
- WO-A1-2017/167512
- WO-A1-2019/145159
- WO-A2-01/36033
- CN-A- 1 285 759
- CN-A- 1 285 759
- CN-A- 1 387 447
- CN-A- 1 387 447
- CN-A- 1 541 125
- CN-A- 104 736 191
- CN-A- 109 718 431
- CN-U- 208 355 880
- DE-U1- 9 204 938
- GB-A- 2 279 879
- US-A- 5 040 527
- US-A1- 2011 011 395
- US-A1- 2016 198 772
- US-B2- 10 098 381

## Description

### TECHNICAL FIELD

The present disclosure relates to the technical field of atomization apparatuses, and in particular, to an electronic atomization device and an air-curtain formation structure used by same.

### BACKGROUND

For an electronic atomization device of related art, such as an e-cigarette or a medical atomizer, condensation is prone to occur when atomizing gas comes into contact with an inner wall of the electronic atomization device. For example, condensate formed on an inner wall of an air outlet channel of the e-cigarette is likely to enter a user's mouth, causing a negative impact on the user experience. For the medical atomizer, and in particular, for a medical atomizer configured to deliver drugs to the lungs, atomizing gas carrying the drugs is condensed in the air outlet channel, causing drug loss. In addition, condensate droplets are easily formed not only in the air outlet channel, but also on other inner walls of the electronic atomization device contacted with the atomizing gas, and consequently, condensate leakage is prone to occur.

US10098381B2 discloses an electronic smoking article. The electronic smoking article includes a heater in communication with a liquid supply reservoir including liquid material and operable to heat the liquid material to a temperature sufficient to volatilize the liquid material contained therein and form an aerosol. The volatilized material flows through a sheath flow and aerosol promoter insert that is operable to cool the aerosol, reduce the particle size of the aerosol and increase the delivery rate of the aerosol.

DE9204938U1 discloses a medical device for inhalation Aerosols. The medical device includes a tubular, preferably cylindrical housing with a recording chamber for aerosol containers, a mouthpiece connected to the housing, and a wall section with a blind bore.

WO2019145159A1 discloses an aerosol generator for an aerosol dispenser. The aerosol generator includes a housing having an inlet part including a liquid inlet configured to guide a liquid jet into the housing and an air inlet configured to guide an air flow into the housing. The housing further having an outlet part including an aerosol outlet configured to guide an aerosol, including liquid mixed with air, out of the housing; wherein the air inlet is configured such that at least part of the air flow entering the housing through the air inlet is obstructed at a distance from the liquid jet entering the housing through the liquid inlet, thereby creating a source of turbulence in the housing to interact with droplets of the liquid jet to prevent coalescence of the droplets.

WO0136033A2 discloses a metered dose inhaler for use with a pressurized aerosol canister. The metered dose inhaler includes a housing defining a conduit with a mouthpiece, and an actuator with a nozzle discharge orifice arranged to discharge aerosol into the conduit. Vortex generators positioned within the wall of the conduit and in fluid communication with air inlets for receiving ambient outside air, provide the inner wall of the conduit with a circumferential-swirling turbulent boundary layer flow to minimize impaction of medication on the inner surfaces of the conduit.

WO2014029827A1 discloses a transition adapter component of a ventilator aerosol delivery system for delivering an aerosol to a patient. The transition adapter component includes a housing having a proximal end and a distal end, the proximal end having an aerosol passage for receiving an aerosol produced by a heated capillary and a gas connection port for receiving carrier gas from a ventilator, which is in communication with a plurality of gas entry ports within the transition adapter. An inner cavity of the transition adapter receives the aerosol from the heated capillary and the streams of carrier gas from a plurality of gas exit ports within the transition adapter and directs the streams of carrier gas at least partially encircling and in parallel with the aerosol. An exit port on the distal end of the transition adapter housing delivers an entrained aerosol to an aerosol delivery connector.

WO2017167512A1 discloses an aerosol-generating system. The aerosol-generating system has a mouth end and a distal end. The system includes a liquid storage portion that has a reservoir containing an aerosol- generating substrate. The system also includes a liquid transfer element to which the aerosol-generating substrate from the reservoir is transferable. The system further includes a power supply and a heating element operably coupled to the power supply and configured to heat the aerosol-generating substrate carried by the transport element to form an aerosol. The system also includes a cover disposed over the liquid storage portion and includes one or more air flow channels between the cover and the liquid storage portion. The system defines an aerosol flow path that extends at least from the liquid transport element to the mouth end of the system. In addition, the system further defines an air flow path through the one or more channels to the mouth end of the system.

WO2015112750A1 discloses methods, devices, systems, and computer readable medium for delivering one or more compounds to a subject. Also described herein are methods, devices, systems, and computer readable medium for transitioning a smoker to an electronic nicotine delivery device and for smoking or nicotine urge relief.

US2016198772A1 discloses a smoking system. The smoking system includes a capillary wick for holding liquid, at least one air inlet, at least one air outlet and a chamber between the air inlet and air outlet. The air inlet, the air outlet and the chamber are arranged so as to define an air flow route from the air inlet to the air outlet via the capillary wick so as to convey aerosol formed from the liquid to the air outlet. The smoking system further includes at least one guide for channeling the air flow in the air flow route, so as to control particle size in the aerosol. The smoking system optionally includes at least one heater for heating the liquid in at least a portion of the capillary wick to form the aerosol.

GB2279879A discloses an inhaler for medicament. The inhaler for medicament includes a housing adapted to receive a pressurised dispensing container of medicament. A mouth piece for insertion into the mouth of a user of the inhaler is connected by duct means to an outlet of the container. Air inlet means are provided for allowing air into the inhaler when a user applies suction to the mouth piece. The air inlet means are provided at a location axially between the air outlet for the duct means and the mouth piece and passage means are provided connecting the inlet to a location adjacent the outlet of the duct means. The arrangement is such that, in use, when a user inhales through the mouth piece an airflow is created from the inlet to the mouth piece and the airflow has a component directed away from the mouth piece towards the outlet of duct means.

CN109718431A discloses a medical care atomizer with improved inner air currents. According to the medical care atomizer disclosed by the application, through a stopping member adopting an inversed truncated cone body structure, a special cavity structure and an outside air entering passage are arranged, so that fog granules with low particle diameter can be effectively screened and enter human bodies, and the atomization efficiency can also be improved.

### SUMMARY

The invention is defined in the appended claims.

In view of the, a technical problem to be mainly resolved in the present disclosure is to provide an electronic atomization device and an air-curtain formation structure applied thereto, to alleviate the problem of atomizing gas condensation. In all aspects, the electronic atomization device and the air-curtain formation structure are provided as set out in the appended set of claims.

Beneficial effects of the present disclosure are as follows: Compared with the related art, the present disclosure provides an electronic atomization device and an air-curtain formation structure used by the same. The air-curtain formation structure has an airflow channel configured to deliver atomizing gas. The airflow channel has a first air inlet channel, and the first air inlet channel is configured to introduce an external airflow into the airflow channel, so that a blocking airflow is formed between an inner wall of the airflow channel and the atomizing gas. In the present disclosure, the blocking airflow is used to block the inner wall of the airflow channel and the atomizing gas, so that the atomizing gas is contacted with the inner wall of the airflow channel as little as possible, the problem of atomizing gas condensation can be alleviated, and less condensate is generated, thereby improving the user experience, reducing drug loss, and reducing the risk of condensate leakage.

### BRIEF DESCRIPTION OF THE DRAWINGS

Accompanying drawings herein are incorporated into the specification and constitute a part of the specification, illustrate embodiments that conform to the present disclosure, and are used for describing a principle of the present disclosure together with the specification. In addition, the accompanying drawings and text descriptions are not intended to limit the scope of the idea of the present disclosure in any form, but to explain the concept of the present disclosure by referring to specific embodiments for a person skilled in the art.
FIG. 1 is a structural schematic view of a first embodiment of an atomization suction nozzle according to the present disclosure.
FIG. 2 is an isometric structural schematic view of a first embodiment of an atomization suction nozzle according to the present disclosure.
FIG. 3 is a structural schematic view of first air inlet channels of an airway body of an atomization suction nozzle according to the present disclosure.
FIG. 4 is a structural schematic view of a second embodiment of an atomization suction nozzle according to the present disclosure.
FIG. 5 is an isometric structural schematic view of a second embodiment of an atomization suction nozzle according to the present disclosure.
FIG. 6 is a bottom view of a second embodiment of an atomization suction nozzle according to the present disclosure.
FIG. 7 is a structural schematic view of a third embodiment of an atomization suction nozzle according to the present disclosure.
FIG. 8 is a cross-sectional structural schematic view of a third embodiment of an atomization suction nozzle according to the present disclosure.
FIG. 9 is a top view of a third embodiment of an atomization suction nozzle according to the present disclosure.
FIG. 10 is a structural schematic view of a first embodiment of an atomizer according to the present disclosure.
FIG. 11 is a partial cross-sectional structural schematic view of a first embodiment of an atomizer according to the present disclosure.
FIG. 12 is a partial cross-sectional structural schematic view of a first embodiment of an atomizer from another perspective according to the present disclosure.
FIG. 13 is a structural schematic view of a fourth embodiment of an atomization suction nozzle according to the present disclosure.
FIG. 14 is a cross-sectional structural schematic view of a fourth embodiment of an atomization suction nozzle according to the present disclosure.
FIG. 15 is a top view of a fourth embodiment of an atomization suction nozzle according to the present disclosure.
FIG. 16 is a structural schematic view of a second embodiment of an atomizer according to the present disclosure.
FIG. 17 is a partial cross-sectional structural schematic view of a second embodiment of an atomizer according to the present disclosure.
FIG. 18 is a structural schematic view of a relative position relationship between a center line of a diverging opening and a joint of a first airflow guide portion and a second airflow guide portion of an atomizer according to the present disclosure.
FIG. 19 is a structural schematic view of a third embodiment of an atomizer according to the present disclosure.
FIG. 20 is a cross-sectional structural schematic view of a third embodiment of an atomizer in a direction A-A according to the present disclosure.
FIG. 21 is a bottom view of a third embodiment of an atomizer according to the present disclosure.
FIG. 22 is a structural schematic view of a fourth embodiment of an atomizer according to the present disclosure.
FIG. 23 is a structural schematic view of an embodiment of an air outlet channel according to the present disclosure.
FIG. 24 is a structural schematic view of an embodiment of an electronic atomization device according to the present disclosure.
FIG. 25 is a structural schematic view of another embodiment of an electronic atomization device according to the present disclosure.
FIG. 26 is a structural schematic view of an embodiment of a medical atomization electronic device according to the present disclosure.
FIG. 27 is a structural schematic view of another embodiment of a medical atomization electronic device according to the present disclosure.

### DETAILED DESCRIPTION OF THE PREFERRED EMBODIMENTS

In order to make the objects, technical solutions, and advantages of the present disclosure clearer, the technical solutions in the embodiments of the present disclosure will be clearly and comprehensively described below with reference to the embodiments of the present disclosure. Apparently, the described embodiments are only a part of the embodiments of the present disclosure, not all of them. All other embodiments obtained by a person of ordinary skill in the art based on the embodiments of the present disclosure without creative efforts shall fall within the protection scope of the present disclosure. The following embodiments and features in the embodiments may be combined with each other in case that no conflict occurs.

To resolve the technical problem of relatively severe atomizing gas condensation in the related art, an embodiment of the present disclosure provides an air-curtain formation structure for an electronic atomization device. The air-curtain formation structure includes an airflow channel configured to deliver atomizing gas. The air-curtain formation structure further includes a first air inlet channel communicated with the airflow channel, and the first air inlet channel is configured to introduce an external airflow into the airflow channel, so that a blocking airflow is formed between an inner wall of the airflow channel and the atomizing gas. Detailed descriptions are provided below.

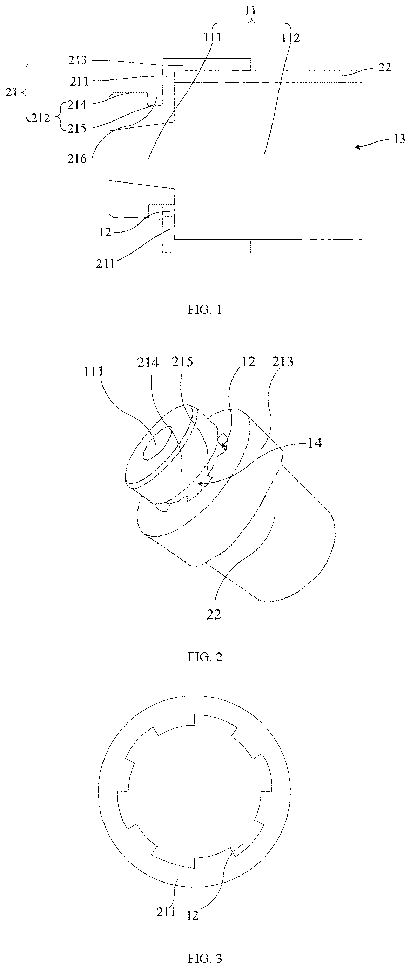

Referring to FIG. 1, FIG. 1 is a structural schematic view of a first embodiment of an atomization suction nozzle according to the present disclosure.

An exemplary embodiment in which the air-curtain formation structure is an atomization suction nozzle for the electronic atomization device is described below.

In the embodiment, the air-curtain formation structure is in a form of the atomization suction nozzle. The atomization suction nozzle provided in the embodiment is for electronic atomization devices such as an e-cigarette and a medical atomizer. Specifically, the atomization suction nozzle has an airflow channel 11. The airflow channel 11 is configured to deliver atomizing gas. The atomization suction nozzle further has a first air inlet channel 12 communicated with the airflow channel 11, and the first air inlet channel 12 is configured to introduce an external airflow into the airflow channel 11, so that a blocking airflow is formed between an inner wall of the airflow channel 11 and the atomizing gas. The blocking airflows form an air curtain.

Further, the atomization suction nozzle further defines an air outlet 13 communicated with the airflow channel 11, the first air inlet channel 12 is close to the inner wall of the airflow channel 11, and an exit of the first air inlet channel 12 faces the air outlet 13, to ensure that the airflow flowing into the airflow channel 11 through the first air inlet channel 12 can flow along the inner wall of the airflow channel 11, that is, the blocking airflow is formed to block the atomizing gas and the inner wall of the airflow channel 11, so that the atomizing gas may be contacted with the inner wall of the airflow channel 11 as little as possible, thereby alleviating the problem of atomizing gas condensation and reducing condensate generation.

Specifically, the atomization suction nozzle includes an airway body 21 and a suction nozzle portion. The suction nozzle portion includes a tube body 22, and the airflow channel 11 is defined in the airway body 21 and the tube body 22. An end of the tube body 22 away from the airway body 21 is the air outlet 13. The first air inlet channel 12 is defined at a position of the airway body 21 close to an inner wall of the tube body 22, to form a blocking airflow between the inner wall of the tube body 22 and the atomizing gas.

The airflow channel 11 includes an entrance channel 111 and an air guide channel 112. The tube body 22 defines the air guide channel 112. The airway body 21 is mounted at one end of the tube body 22, the airway body 21 defines the entrance channel 111, and the entrance channel 111 of the airway body 21 is communicated with the air guide channel 112 of the tube body 22. The entrance channel 111 is configured to introduce the atomizing gas and deliver the atomizing gas into the air guide channel 112.

Referring to FIG. 1 and FIG. 2, FIG. 2 is an isometric structural schematic view of a first embodiment of an atomization suction nozzle according to the present disclosure. When the airway body 21 is mounted at one end of the tube body 22, a part of the airway body 21 abuts against an end of the air guide channel 112 and covers a part of the air guide channel 112. The first air inlet channel 12 communicated with the air guide channel 112 is provided at a position where the airway body 21 covers the air guide channel 112. Alternatively, the airway body 21 includes a wall portion 211 abutting against one end of the air guide channel 112 and covering the part of the air guide channel 112, and the first air inlet channel 12 is opened in the wall portion 211 and is communicated with the air guide channel 112.

Condensate is easily formed on an inner wall of the air guide channel 112 due to moisture in the atomizing gas. The first air inlet channel 12 is provided, and air is introduced into the first air inlet channel 12. When suction is performed on the atomization suction nozzle, that is, the atomizing gas is suctiond from the end of the tube body 22 away from the airway body 21, an air pressure difference is formed inside the atomization suction nozzle, so that under the action of the air pressure difference, air entering through the first air inlet channel 12 is adhered to the inner wall of the air guide channel 112 and forms blocking airflows on the inner wall of the air guide channel 112 to block the atomizing gas and the inner wall of the air guide channel 112, thereby reducing condensate formed by the atomizing gas on the inner wall of the air guide channel 112. When suction is not performed on the atomization suction nozzle, there is no air pressure difference inside the atomization suction nozzle, and there is no blocking airflow formed on the inner wall of the air guide channel 112.

Further, a flow direction of the blocking airflows is parallel to the inner wall of the airflow channel 11, that is, the flow direction of the blocking airflows is parallel to the inner wall of the air guide channel 112, and to be specific, the flow direction of the blocking airflows is parallel to the inner wall of the tube body 22, to ensure a desirable effect of the blocking airflows for blocking the atomizing gas and the inner wall of the tube body 22.

Alternatively, in order to enable the blocking airflows to be adhered to the inner wall of the air guide channel 112 to form an air curtain, in a specific embodiment, there may be a plurality of first air inlet channels 12, and the plurality of first air inlet channels 12 are spaced in a circumferential direction of the wall portion 211.

Referring to FIG. 3 together, FIG. 3 is a structural schematic view of first air inlet channels of an airway body of an atomization suction nozzle according to the present disclosure. The airway body 21 includes a wall portion 211 covering the air guide channel 112. The first air inlet channels 12 are defined on the wall portion 211 and are communicated with the air guide channel 112. As shown in FIG. 3, there are a plurality of first air inlet channels 12 evenly arranged along the wall portion 211 in a circumferential direction. In a specific embodiment, the shape of the first air inlet channel 12 is not limited as long as air can flow into the air guide channel 112 through the first air inlet channels 12 during suction. In an alternative embodiment, the shape of the first air inlet channel 12 may be any one of or any combination of a square, a circle, or a triangle.

Further, the size of the first air inlet channel 12 is to be appropriately set when the first air inlet channels 12 are provided, so that air entering through the first air inlet channel 12 can form blocking airflows completely covering the inner wall of the air guide channel 112 on the inner wall of the air guide channel 112.

Still referring to FIG. 1, the airway body 21 of the atomization suction nozzle in the present disclosure includes a first airway portion 212, a second airway portion 213, and the wall portion 211 connecting the first airway portion 212 and the second airway portion 213 and covering a part of the air guide channel 112. The entrance channel 111 is mainly defined in the first airway portion 212 and is communicated with the air guide channel 112, and the second airway portion 213 is sleeved on an outer side of the tube body 22 of the suction nozzle portion. In a specific embodiment, the wall portion 211 abuts against one end of the air guide channel 112. In another embodiment, there may be a gap between the wall portion 211 and one end of the air guide channel 112 as long as it can be ensured that atomizing gas will not leak out.

Alternatively, still referring to FIG. 2, the first airway portion 212 further includes a vent portion 214 and a first connection portion 215, the first connection portion 215 is arranged on one side of the wall portion 211 away from the second airway portion 213, and the vent portion 214 is arranged on one side of the first connection portion 215 away from the wall portion 211. A cross-sectional area of the first connection portion 215 is less than a cross-sectional area of the vent portion 214, and the cross section is defined as a section perpendicular to an axial direction, similarly hereinafter. A clamping opening 216 is formed at a position where the first connection portion 215 is connected to the vent portion 214, and the clamping opening 216 is configured to clamp an atomizing gas generation device (i.e a part of the electronic atomization device for generating atomizing gas, not shown in the figure). Further, referring to FIG. 1 and FIG. 2, a comprehensive airway 14 is formed at the clamping opening 216, so that air flows into the first air inlet channels 12 through the comprehensive airway 14 and then flows into the air guide channel 112 through the first air inlet channels 12. During suction, after the air pressure difference is generated, blocking airflows are formed on the inner wall of the air guide channel 112 under the action of the air pressure difference. The blocking airflows block the atomizing gas and the air guide channel 112, thereby reducing condensate formed by the atomizing gas in the air guide channel 112.

Alternatively, in an implementation, the first airway portion 212, the second airway portion 213, and the wall portion 211 connecting the first airway portion 212 and the second airway portion 213 of the airway body 21 are integrally formed. In another implementation, the first airway portion 212, the second airway portion 213, and the wall portion 211 connecting the first airway portion 212 and the second airway portion 213 of the airway body 21 may also be formed through a welding process.

Alternatively, the second airway portion 213 of the airway body 21 is sleeved on the outer side of the tube body 22 of the suction nozzle portion. Specifically, in an implementation, the airway body 21 and the tube body 22 may be designed to be integrally formed. In another implementation, the second airway portion 213 may alternatively be sleeved on the outer side of the tube body 22 of the suction nozzle portion in a matching manner. In order to avoid atomizing gas from leaking out, the second airway portion 213 may be sleeved on the outer side of the tube body 22 of the suction nozzle portion in an interference-fitting manner.

In the atomization suction nozzle provided in the embodiment, the first air inlet channels 12 communicated with the air guide channel 112 are defined on the wall portion 211 covering the air guide channel 112. When a suction action is performed on the tube body 22 and the atomizing gas flows into the air guide channel 112 through the entrance channel 111, and air flows into the air guide channel 112 through the first air inlet channels 12. The air entering through the first air inlet channels 12 may form blocking airflows on the inner wall of the air guide channel 112 under the action of the air pressure, to block the atomizing gas and the inner wall of the air guide channel 112, thereby preventing the atomizing gas from forming condensate on the inner wall of the air guide channel 112.

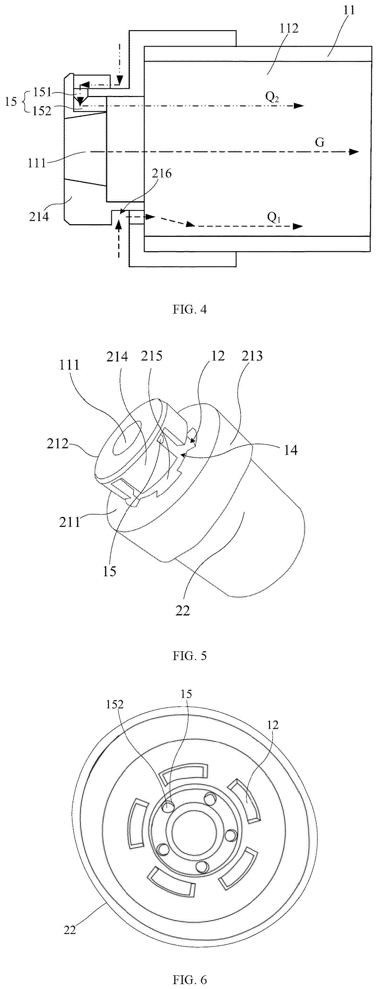

Referring to FIG. 4, FIG. 4 is a structural schematic view of a second embodiment of an atomization suction nozzle according to the present disclosure. Compared with the first embodiment shown in FIG. 1, the difference between the first embodiment and the second embodiment is that: in the embodiment, second air inlet channels 15 are defined on an outer side of the vent portion 214. The second air inlet channels 15 are configured to increase a speed of discharging the atomizing gas, to further prevent the atomizing gas from forming condensate on the inner wall of the air guide channel 112.

Alternatively, the second air inlet channel 15 has an air inlet portion 151 and an air guide portion 152. Specifically, the air inlet portion 151 is arranged surrounding the vent portion 214 in a direction parallel to the wall portion 211, an extending direction of the air guide portion 152 is parallel to an extending direction of the entrance channel 111, and the air guide portion 152 is connected to an end of the air inlet portion 151 arranged in the vent portion 214. Air enters through the air inlet portion 151 and flows into the air guide channel 112 through the air guide portion 152.

Alternatively, referring to FIG. 4 and FIG. 5, FIG. 5 is an isometric structural schematic view of a second embodiment of an atomization suction nozzle according to the present disclosure. The second air inlet channels 15 are defined in the vent portion 214, and the first air inlet channels 12 are defined on the wall portion 211 connecting the first airway portion 212 and the second airway portion 213. In a specific embodiment, when there is a suction force in the tube body 22 of the suction nozzle portion, the atomizing gas enters through the entrance channel 111, and air enters through the second air inlet channels 15 and the first air inlet channels 12. Referring to FIG. 6, FIG. 6 is a bottom view of a second embodiment of an atomization suction nozzle according to the present disclosure. As shown in FIG. 6, the first air inlet channels 12 are closer to the inner wall of the tube body 22 relative to the air guide portions 152 in the second air inlet channels 15. Therefore, during suction, air enters through the first air inlet channels 12 and forms blocking airflows on the inner wall of the air guide channel of the tube body 22 of the suction nozzle portion under the action of the air pressure, to block the atomizing gas entering through the entrance channel 111 and the inner wall of the air guide channel, thereby reducing condensate formed by the atomizing gas on the inner wall of the air guide channel. Further, the second air inlet channels 15 are provided, air flows into the second air inlet channels 15 during suction, and the air increases the speed of discharging the atomizing gas entering through the entrance channel 111 from the air guide channel 112, thereby further preventing the atomizing gas from forming condensate on the inner wall of the air guide channel.

Further, still referring to FIG. 4 and FIG. 5, a clamping opening 216 is formed at a position where the first connection portion 215 is connected to the vent portion 214, and the clamping opening 216 is configured to clamp an atomizing gas generation device (not shown in the figure). Further, a comprehensive airway 14 is formed at a position of the clamping opening 216, so that air flows into the first air inlet channels 12 and the second air inlet channels 15 through the comprehensive airway 14 and then flows into the air guide channel 112 through the first air inlet channels 12. During suction, after the air pressure difference is generated, blocking airflows are formed on the inner wall of the air guide channel 112 under the action of the air pressure difference. As shown in FIG. 4, the blocking airflow (as shown by an arrow Q1 in FIG. 4) blocks the atomizing gas (as shown by an arrow G in FIG. 4) and the air guide channel 112, thereby reducing condensate formed by the atomizing gas on the inner wall of the air guide channel 112. Air flows into the air guide portions 152 through the second air inlet channels 15 and forms a second airflow after flowing into the air guide channel 112. The second airflow increases the speed of discharging the atomizing gas.

Alternatively, in the embodiment, the shape of the air guide portion 152 of the second air inlet channel 15 may be any one of or any combination of a square, a circle, or a triangle. The shape of the air inlet portion 151 of the second air inlet channel 15 may alternatively be any one of or any combination of a square, a circle, or a triangle, which is not specifically limited as long as air can be introduced into the air guide portion 152 and then flows into the air guide channel 112.

In an embodiment, there is at least one second air inlet channel 15 circumferentially provided on an outer side of the vent portion 214.

In an embodiment, the first air inlet channels 15 may be provided corresponding to the second air inlet channels 12. In another embodiment, the second air inlet channels 15 and the first air inlet channels 12 may also be staggered. Specifically, in order to reduce the mutual impact between airflows formed by air flowing into the first air inlet channels 12 and the second air inlet channels 15, the second air inlet channels 15 and the first air inlet channels 12 are staggered, as shown in FIG. 6.

In an embodiment, when the first air inlet channels 12 and the second air inlet channels 15 are provided, there is a speed difference between the airflows formed in the second air inlet channels 15 and the first air inlet channels 12, to ensure that air entering through the first air inlet channels 12 forms blocking airflows on the inner wall of the air guide channel 112, thereby blocking the atomizing gas and the air guide channel 112, and and realizing air entering through the second air inlet channels 15 can increase the speed of discharging the atomizing gas. In a specific embodiment, a flow rate of the airflows formed in the first air inlet channels 12 is greater than a flow rate of the airflows formed in the second air inlet channels 15, thereby weakening the impact on a deliver direction of the atomizing gas while achieving the effect of reducing the condensate.

Specifically, the flow rates of the airflows formed in the first air inlet channel 12 and the second air inlet channel 15 are related to the size of the opening. A larger size of the opening indicates a faster flow rate. Therefore, in a specific implementation, in order to realize that the flow rate of the airflow formed in the first air inlet channel 12 is greater than the flow rate of the airflow formed in the second air inlet channel 15, a size of the first air inlet channel 12 (that is, a cross-sectional area, where a cross section of the first air inlet channel 12 should be a section taken perpendicular to an extending direction of the first air inlet channel 12) is set to be greater than a size of the second air inlet channel 15 (that is, a cross-sectional area, where a cross section of the second air inlet channel 15 should be a section taken perpendicular to an extending direction of the second air inlet channel 15). Alternatively, in another embodiment, a quantity of the first air inlet channels 12 is greater than a quantity of the second air inlet channels 15.

As shown in FIG. 4, when an air pressure difference is generated because a user suctions, under the action of the air pressure difference, an external airflow flows into the airflow channel 11 (that is, the air guide channel 112) through the first air inlet channel 12 and then forms a blocking airflow Q1 on the inner wall of the airflow channel 11. The blocking airflow Q1 blocks atomizing gas G and the inner wall of the airflow channel 11, thereby reducing condensate formed by the atomizing gas G on the inner wall of the airflow channel 11. **In** addition, an airflow Q2 flowing into the airflow channel 11 through the second air inlet channel 15 flows along an outer edge of the atomizing gas G under the action of the air pressure difference, thereby increasing the speed of discharging the atomizing gas G.

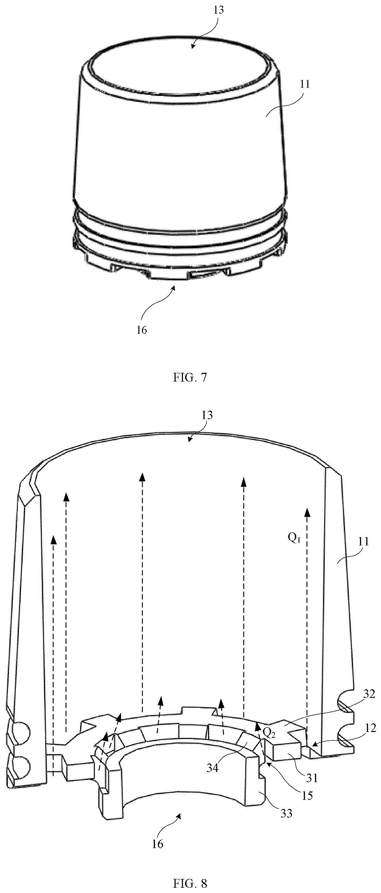

Referring to FIG. 7 and FIG. 8, FIG. 7 is a structural schematic view of a third embodiment of an atomization suction nozzle according to the present disclosure, and FIG. 8 is a cross-sectional structural schematic view of a third embodiment of an atomization suction nozzle according to the present disclosure.

An exemplary embodiment in which the air-curtain formation structure is an atomization suction nozzle for the electronic atomization device is described below.

In the embodiment, the air-curtain formation structure is in a form of the atomization suction nozzle. The atomization suction nozzle provided in the embodiment is for electronic atomization devices such as an e-cigarette and a medical atomization suction nozzle.

Specifically, the atomization suction nozzle has an airflow channel 11 configured to deliver atomizing gas. The atomization suction nozzle further has first air inlet channels 12 communicated with the airflow channel 11, and the first air inlet channels 12 are configured to introduce external airflows into the airflow channel 11, so that blocking airflows (as shown by arrows Q1 in FIG. 8, similarly hereinafter) are formed between the inner wall of the airflow channel 11 and the atomizing gas. The blocking airflows form an air curtain.

Further, the atomization suction nozzle further has a first air inlet 16 and an air outlet 13. The first air inlet 16 and the air outlet 13 are provided opposite to each other and respectively communicated with the airflow channel 11. Atomizing gas flows into the airflow channel 11 through the first air inlet 16 and is delivered to the air outlet 13 through the airflow channel 11, and then the atomizing gas is output from the air outlet 13 for the user to suction. The first air inlet channels 12 are close to the inner wall of the airflow channel 11, and exits of the first air inlet channels 12 face the air outlet 13, to ensure that the airflows flowing into the airflow channel 11 through the first air inlet channels 12 can flow along the inner wall of the airflow channel 11 (i.e, an inner wall of the atomization suction nozzle), that is, the blocking airflows (as shown by arrows Q1 in FIG. 8, similarly hereinafter) are formed to block the atomizing gas and the inner wall of the airflow channel 11, that is, block the atomizing gas and the inner wall of the atomization suction nozzle, so that the atomizing gas may be contacted with the inner wall of the atomization suction nozzle as little as possible, thereby alleviating the problem of atomizing gas condensation and reducing condensate generation.

Further, a flow direction of the blocking airflows is parallel to the inner wall of the airflow channel 11, that is, the flow direction of the blocking airflows is parallel to the inner wall of the atomization suction nozzle, to ensure a desirable effect of the blocking airflows blocking the atomizing gas and the inner wall of the atomization suction nozzle.

**In** an embodiment, still referring to FIG. 8, the atomization suction nozzle further includes a first airflow guide portion 31. The first air inlet channels 12 are formed between the first airflow guide portion 31 and the inner wall of the airflow channel 11, and configured to guide airflows introduced through the first air inlet channels 12 to flow along the inner wall of the airflow channel 11, to form the blocking airflows.

Further, the atomization suction nozzle further includes a second connection portion 32. The first airflow guide portion 31 is connected to the inner wall of the airflow channel 11 through the second connection portion 32.

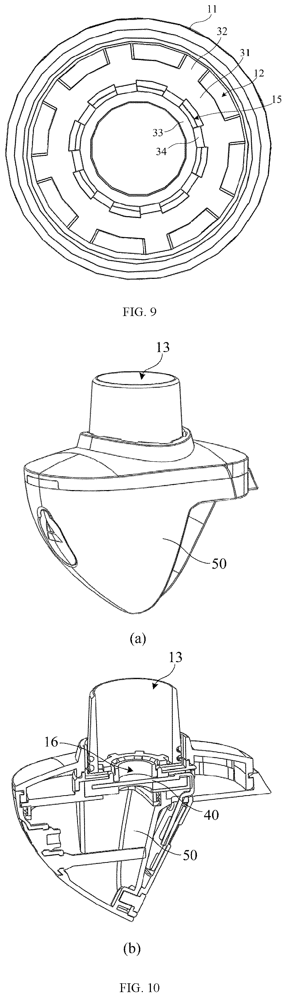

Specifically, referring to FIG. 9 together, a plurality of second connection portions 32 are arranged between the first airflow guide portion 31 and the inner wall of the airflow channel 11. The plurality of second connection portions 32 are spaced along a circumferential direction of the first airflow guide portion 31, and the first air inlet channel 12 is formed between adjacent second connection portions 32, that is, at least one first air inlet channel 12 is formed. In the way, a relative position of the first airflow guide portion 31 in the atomization suction nozzle is fixed, and formation of the first air inlet channels 12 between the first airflow guide portion 31 and the inner wall of the airflow channel 11 is ensured.

Further, a plurality of first air inlet channels 12 can be formed between the first airflow guide portion 31 and the inner wall of the airflow channel 11, blocking airflows formed by the plurality of first air inlet channels 12 form an air curtain, as shown in FIG. 8, thereby greatly enabling the atomizing gas to be contacted with the inner wall of the atomization suction nozzle (that is, the inner wall of the airflow channel 11) as little as possible, alleviating the problem of atomizing gas condensation, and reducing condensate generation.

Alternatively, the first airflow guide portion 31 may be in an annular shape corresponding to an inner space of the atomization suction nozzle, and surrounds along a circumferential direction of the atomization suction nozzle.

**In** an embodiment, still referring to FIG. 8, the atomization suction nozzle further includes a second airflow guide portion 33. The second airflow guide portion 33 is away from the inner wall of the airflow channel 11 relative to the first airflow guide portion 31, the second air inlet channels 15 are formed between the second airflow guide portion 33 and the first airflow guide portion 31, the exits of the second air inlet channels 15 face the air outlet 13, and airflows (as shown by arrows Q2 in FIG. 8) entering through the second air inlet channels 15 are used to guide the atomizing gas to be output from the air outlet 13, thereby speeding up the discharge of the atomizing gas.

Further, the second airflow guide portion 33 is annularly arranged and surrounds the first air inlet 16 of the atomization suction nozzle.

Further, the air-curtain formation structure further includes a third connection portion 34, and the second airflow guide portion 33 is connected to the first airflow guide portion 31 through the third connection portion 34, so that a relative position of the second airflow guide portion 33 in the atomization suction nozzle is fixed through the first airflow guide portion 31.

Specifically, referring to FIG. 9 together, a plurality of third connection portions 34 are arranged between the second airflow guide portion 33 and the first airflow guide portion 31, the plurality of third connection portions 34 are sequentially spaced along a circumferential direction of the second airflow guide portion 33, and the second air inlet channels 15 are formed between adjacent third connection portions 34. In the way, the relative position of the second airflow guide portion 33 in the atomization suction nozzle is fixed, and formation of the second air inlet channels 15 between the second airflow guide portion 33 and the first airflow guide portion 31 is ensured.

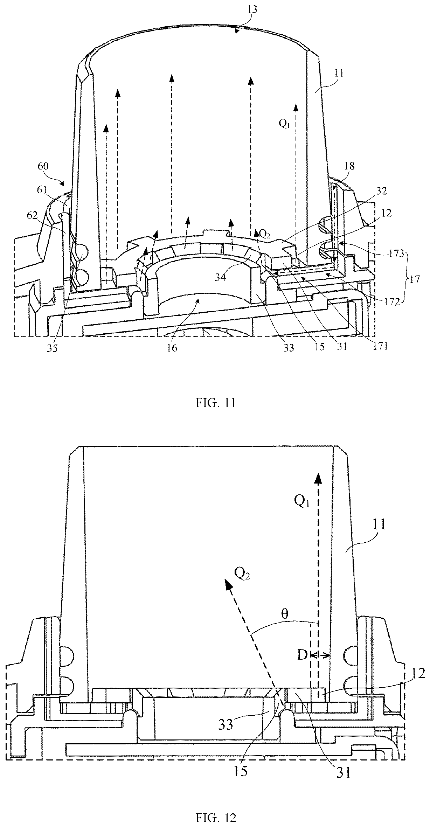

Referring to FIG. 10 and FIG. 11, FIG. 10 is a structural schematic view of a first embodiment of an atomizer according to the present disclosure, and FIG. 11 is a partial cross-sectional structural schematic viewof a first embodiment of an atomizer according to the present disclosure.

An exemplary embodiment in which the air-curtain formation structure is an atomizer for the electronic atomization device is described below.

In the embodiment, the air-curtain formation structure is in a form of the atomizer. The atomizer provided in the embodiment is for an electronic atomization device such as an e-cigarette and a medical atomizer. FIG. 10 shows a case in which the air-curtain formation structure is for the medical atomizer, which is merely used for description and is not intended to limit an application environment of the air-curtain formation structure in the embodiment.

In the embodiment, referring to FIG. 10, the air-curtain formation structure includes an atomization suction nozzle, an atomization core 40, and a liquid storage cavity 50. The atomization suction nozzle defines a first air inlet 16 and an air outlet 13. Atomizing gas flows into the atomization suction nozzle through the first air inlet 16 and is delivered to the air outlet 13 through the atomization suction nozzle, and then is output from the air outlet 13 for the user to suction. The atomization core 40 is arranged at a position of the first air inlet 16 of the atomization suction nozzle, and is configured to atomize an aerosol generation substrate stored in the liquid storage cavity 50 to generate atomizing gas. Structures such as the atomization core 40, the liquid storage cavity 50 and the like constitute atomizing gas generation device of the air-curtain formation structure in the embodiment configured to generate atomizing gas.

For the case in which the air-curtain formation structure in the embodiment is for the medical atomizer, the atomization core 40 may be an ultrasonic atomization sheet and the like, and the ultrasonic atomization sheet atomizes the aerosol generation substrate through high-frequency oscillation. The specific principle thereof falls within the understanding scope of a person skilled in the art, and details are not described herein again. Certainly, for the case in which the air-curtain formation structure is for other fields, the atomization core 40 may also generate atomizing gas in a manner of heating and atomizing the aerosol generation substrate, which is not limited herein.

Specifically, referring to FIG. 11 together, the atomization suction nozzle defines an airflow channel 11. The airflow channel 11 is configured to deliver atomizing gas. The atomization suction nozzle further includes first air inlet channels 12 communicated with the airflow channel 11, and are configured to introduce external airflows into the airflow channel 11, so that blocking airflows (as shown by arrows Q1 in FIG. 11, similarly hereinafter) are formed between the inner wall of the airflow channel 11 and the atomizing gas. The blocking airflows form an air curtain.

Further, the first air inlet 16 and the air outlet 13 are provided opposite to each other and are respectively communicated with the airflow channel 11. The first air inlet channels 12 are close to the inner wall of the airflow channel 11, and exits of the first air inlet channels 12 face the air outlet 13, to ensure that the airflows flowing into the airflow channel 11 through the first air inlet channels 12 can flow along the inner wall of the airflow channel 11 (i.e, an inner wall of the atomization suction nozzle), that is, the blocking airflows are formed to block the atomizing gas and the inner wall of the airflow channel 11, that is, block the atomizing gas and the inner wall of the atomization suction nozzle, so that the atomizing gas may be contacted with the inner wall of the atomization suction nozzle as little as possible, thereby alleviating the problem of atomizing gas condensation and reducing condensate generation.

Further, a flow direction of the blocking airflows is parallel to the inner wall of the airflow channel 11, that is, the flow direction of the blocking airflows is parallel to the inner wall of the atomization suction nozzle, to ensure a desirable effect of the blocking airflows blocking the atomizing gas and the inner wall of the atomization suction nozzle.

**In** an embodiment, still referring to FIG. 11, the atomization suction nozzle further includes a first airflow guide portion 31. The first air inlet channels 12 are formed between the first airflow guide portion 31 and the inner wall of the airflow channel 11, and are configured to guide airflows introduced through the first air inlet channels 12 to flow along the inner wall of the airflow channel 11, to form the blocking airflows.

Further, the atomization suction nozzle further includes a second connection portion 32. The first airflow guide portion 31 is connected to the inner wall of the airflow channel 11 through the second connection portion 32.

Specifically, a plurality of second connection portions 32 are arranged between the first airflow guide portion 31 and the inner wall of the airflow channel 11. The plurality of second connection portions 32 are spaced along a circumferential direction of the first airflow guide portion 31, and the first air inlet channel 12 is formed between adjacent second connection portions 32, that is, at least one first air inlet channel 12 is formed. In the way, a relative position of the first airflow guide portion 31 in the atomization suction nozzle is fixed, and formation of the first air inlet channels 12 between the first airflow guide portion 31 and the inner wall of the airflow channel 11 is ensured.

Alternatively, the first airflow guide portion 31 may be in an annular shape corresponding to an inner space of the atomization suction nozzle, and surrounds along a circumferential direction of the atomization suction nozzle.

In an embodiment, still referring to FIG. 11, the atomization suction nozzle further includes a second airflow guide portion 33. The second airflow guide portion 33 is away from the inner wall of the airflow channel 11 relative to the first airflow guide portion 31, the second air inlet channels 15 are formed between the second airflow guide portion 33 and the first airflow guide portion 31, the exits of the second air inlet channels 15 face the air outlet 13, and airflows entering through the second air inlet channels 15 are used to guide the atomizing gas to be output from the air outlet 13, thereby speeding up the discharge of the atomizing gas.

Further, the second airflow guide portion 33 is annularly arranged and surrounds the first air inlet 16 of the atomization suction nozzle.

Further, the air-curtain formation structure further includes a third connection portion 34, and the second airflow guide portion 33 is connected to the first airflow guide portion 31 through the third connection portion 34, so that a relative position of the second airflow guide portion 33 in the atomization suction nozzle is fixed through the first airflow guide portion 31.

Specifically, a plurality of third connection portions 34 are arranged between the second airflow guide portion 33 and the first airflow guide portion 31, the plurality of third connection portions 34 are sequentially spaced along a circumferential direction of the second airflow guide portion 33, and the second air inlet channels 15 are formed between adjacent third connection portions 34. In the way, the relative position of the second airflow guide portion 33 in the atomization suction nozzle is fixed, and formation of the second air inlet channels 15 between the second airflow guide portion 33 and the first airflow guide portion 31 is ensured.

In an embodiment, still referring to FIG. 11, the air-curtain formation structure further defines a converging channel 17, one end of the converging channel 17 is an air inlet, that is, a second air inlet 18, and the other end of the converging channel 17 is a diverging opening 171, and the diverging opening 171 is respectively communicated with the first air inlet channels 12 and the second air inlet channels 15.

Specifically, the converging channel 17 has a first channel section 172 and a second channel section 173 communicated with each other, an end opening of the first channel section 172 away from the second channel section 173 is the diverging opening 171, and an end opening of the second channel section 173 away from the first channel section 172 is the air inlet, that is, the second air inlet 18. An extending direction of the first channel section 172 is different from an extending direction of the second channel section 173.

FIG. 11 shows that the extending direction of the first channel section 172 is a horizontal direction, the extending direction of the second channel section 173 is a vertical direction, and the second channel section 173 extends toward the air outlet 13. When the user suctions, an external airflow flows into the second channel section 173 through the second air inlet 18 and is delivered into the first channel section 172, and then passes through the diverging opening 171 and flows into the airflow channel 11 in the atomization suction nozzle respectively through the first air inlet channels 12 and the second air inlet channels 15. Flow of airflows is shown by dashed arrows in FIG. 11.

Further, the air-curtain formation structure includes a mounting portion 60. The mounting portion 60 includes a mounting protrusion 61 and a vent groove 62 The mounting protrusion 61 is configured to fix the atomization suction nozzle. After the atomization suction nozzle is fixed to the mounting portion 60, the first channel section 172 is formed between the atomization suction nozzle and the mounting portion 60, and to be specific, the first channel section 172 is formed between the atomization suction nozzle and the bottom of the mounting portion 60. In addition, the second channel section 173 is formed between the vent groove 62 and the atomization suction nozzle.

In an embodiment, still referring to FIG. 11, the periphery of the atomization suction nozzle is provided with limiting grooves 35 surrounding along a circumferential direction thereof, and the limiting grooves 35 are configured to place elastic rings to fix the atomization suction nozzle. Specifically, after the atomization suction nozzle is embedded in the mounting portion 60 mentioned above, the elastic rings placed in the limiting grooves 35 are in elastically interference fit with the mounting protrusion 61 in the mounting portion 60, to fix the atomization suction nozzle in the mounting portion 60.

It is to be noted that, the elastic ring arranged at a position of the vent groove 62 in the mounting portion 60 is arranged may not block a gap between the atomization suction nozzle and the vent groove 62, to ensure a ventilation function between the atomization suction nozzle and the vent groove 62, thereby ensuring that the external airflows can flow into the airflow channel 11 to form blocking airflows and speed up the discharge of the atomizing gas.

Alternatively, there may be a plurality of limiting grooves 35 spaced in an axial direction of the atomization suction nozzle. The design of the plurality of limiting grooves 35 can ensure the sufficient bonding strength between the atomization suction nozzle and the mounting portion 60, prevent the atomization suction nozzle from falling off. In addition, the elastic ring may be a silicone ring, which is not limited herein.

Referring to FIG. 12, FIG. 12 is a partial cross-sectional structural schematic view of a first embodiment of an atomizer from another perspective according to the present disclosure. Airflow in the first air inlet channel 12 and the second air inlet channel 15 in the exemplary embodiment is described below.

According to an aspect, a cross-sectional area of the first air inlet channel 12 may affect an amount of the blocking airflows. Specifically, in a case that the air pressure difference caused by user suctioning is fixed, within a specific range, a larger a cross-sectional area of the first air inlet channel 12 indicates a larger amount of the blocking airflows. To be specific, a larger distance D between the first airflow guide portion 31 and the inner wall of the atomization suction nozzle (i.e, the inner wall of the airflow channel 11) indicates a larger cross-sectional area of the first air inlet channel 12 and a larger amount of the blocking airflows.

It may be understood that, since the air pressure difference caused by user suctioning is limited, there is an upper limit on the amount of the blocking airflows. When the amount of the blocking airflows reaches the upper limit, the amount of the blocking airflows may not significantly increase even if the distance between the first airflow guide portion 31 and the inner wall of the atomization suction nozzle continues to be increased.

According to another aspect, a flow direction of an airflow (as shown by an arrow Q2 in FIG. 12, similarly hereinafter) flowing into the airflow channel 11 (i.e, the atomization suction nozzle) through the second air inlet channel 15 may affect airflow in the airflow channel 11. Specifically, when an angle (as shown by an angle θ in FIG. 12, similarly hereinafter) between the flow direction of the airflow entering through the second air inlet channel 15 and a preset direction is excessively small, the airflow entering through the second air inlet channel 15 is affected and drawn by the blocking airflow. As a result, the airflow entering through the second air inlet channel 15 cannot be output well carrying the atomizing gas, and the effect of speeding up the discharge of the atomizing gas is greatly weakened. When the angle between the flow direction of the airflow entering through the second air inlet channel 15 and the preset direction is excessively large, the airflow entering through the second air inlet channel 15 may block an output path of the atomizing gas, and prevents the atomizing gas from being delivered to the air outlet 13 of the atomization suction nozzle. The preset direction is parallel to a flow direction of the blocking airflow (as shown by an arrow Q1 in FIG. 12), that is, the preset direction may be represented by the flow direction of the blocking airflow.

In view of the above, the angle between the flow direction of the airflow entering through the second air inlet channel 15 and the preset direction preferably ranges from 30° to 45°, for example, 30°, 33°, 37°, 41°, 43°, 45°, or the like. In this way, it can be ensured that the airflow entering through the second air inlet channel 15 can be output carrying the atomizing gas, to speed up the discharge of the atomizing gas.

It is to be noted that, the flow direction of the airflow entering through the second air inlet channel 15 can be adjusted by adjusting the structure of the atomization suction nozzle at a position of the second air inlet channel 15. For example, the flow direction of the airflow entering through the second air inlet channel 15 can be adjusted by adjusting positions of the first airflow guide portion 31 and the second airflow guide portion 33 in an axial direction of the airflow channel 11, which is not limited herein.

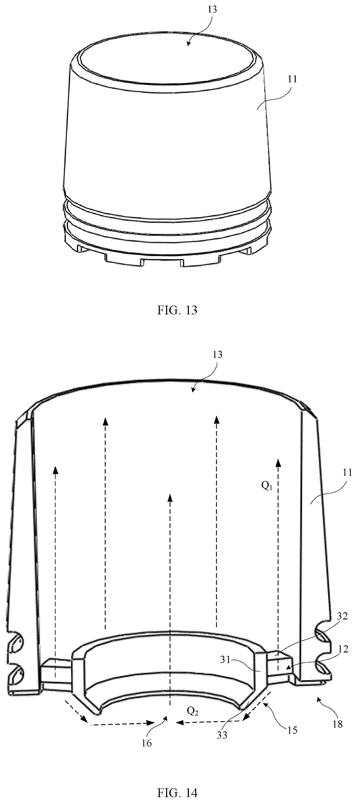

Referring to FIG. 13 and FIG. 14, FIG. 13 is a structural schematic view of a fourth embodiment of an atomization suction nozzle according to the present disclosure, and FIG. 14 is a cross-sectional structural schematic view of a fourth embodiment of an atomization suction nozzle according to the present disclosure.

An exemplary embodiment in which the air-curtain formation structure is an atomization suction nozzle for the electronic atomization device is described below. The atomization suction nozzle defines a first air inlet 16, a second air inlet 18, and an air outlet 13, and the first air inlet 16 and the air outlet 13 are provided opposite to each other. The atomization suction nozzle further includes an airflow guide member. The airflow guide member is communicated with the second air inlet 18 and is configured to guide an airflow entering through the second air inlet 18 toward the first air inlet 16. Detailed descriptions are provided below.

In the embodiment, the air-curtain formation structure is in a form of the atomization suction nozzle. The atomization suction nozzle provided in the embodiment is for electronic atomization devices such as an e-cigarette and a medical atomization suction nozzle.

Specifically, referring to FIG. 14, the atomization suction nozzle defines an airflow channel 11. The airflow channel 11 is configured to deliver atomizing gas. The atomization suction nozzle further includes first air inlet channels 12 communicated with the airflow channel 11, and the first air inlet channels 12 are configured to introduce external airflows into the airflow channel 11, so that blocking airflows (as shown by arrows Q1 in FIG. 11, similarly hereinafter) are formed between the inner wall of the airflow channel 14 and the atomizing gas. The blocking airflows form an air curtain.

Further, the atomization suction nozzle is further provided with a first air inlet 16 and an air outlet 13. The first air inlet 16 and the air outlet 13 are provided opposite to each other and respectively communicated with the airflow channel 11. Atomizing gas flows into the airflow channel 11 through the first air inlet 16 and is delivered to the air outlet 13 through the airflow channel 11, and then is output from the air outlet 13 for the user to suction. The first air inlet channels 12 are close to the inner wall of the airflow channel 11, and exits of the first air inlet channels 12 face the air outlet 13, to ensure that the airflows flowing into the airflow channel 11 through the first air inlet channels 12 can flow along the inner wall of the airflow channel 11 (i.e, an inner wall of the atomization suction nozzle), that is, the blocking airflows are formed to block the atomizing gas and the inner wall of the airflow channel 11, that is, block the atomizing gas and the inner wall of the atomization suction nozzle, so that the atomizing gas may be contacted with the inner wall of the atomization suction nozzle as little as possible, thereby alleviating the problem of atomizing gas condensation and reducing condensate generation.

Further, a flow direction of the blocking airflows is parallel to the inner wall of the airflow channel 11, that is, the flow direction of the blocking airflows is parallel to the inner wall of the atomization suction nozzle, to ensure a desirable effect of the blocking airflows blocking the atomizing gas and the inner wall of the atomization suction nozzle.

In an embodiment, still referring to FIG. 14, the atomization suction nozzle is further provided with the second air inlet 18 different from the first air inlet 16, and the second air inlet 18 is configured to guide an external airflow to flow into the atomization suction nozzle. The atomization suction nozzle further includes an airflow guide member. The airflow guide member is communicated with the second air inlet 18 and is configured to guide the airflow entering through the second air inlet 18 to flow toward the first air inlet 16, and then carry atomizing gas flowing into the atomization suction nozzle through the first air inlet 16 and output the atomizing gas through the air outlet 13 of the atomization suction nozzle, so that the user can suction and the discharge of the atomizing gas can be accelerated.

Specifically, at least a part of the airflow guide member is obliquely arranged in a direction away from the inner wall (i.e, the inner wall of the airflow channel 11) and the air outlet 13 of the atomization suction nozzle, to guide the airflow entering through the second air inlet 18 to flow toward the first air inlet 16, and further carry the atomizing gas flowing into the atomization suction nozzle through the first air inlet 16 and output the atomizing gas through the air outlet 13 of the atomization suction nozzle, so that the user can suction and the discharge of the atomizing gas can be accelerated.

In an embodiment, the airflow guide member includes a first airflow guide portion 31. The first air inlet channels 12 are formed between the first airflow guide portion 31 and the inner wall of the airflow channel 11 (i.e, the inner wall of the atomization suction nozzle), and are configured to guide airflows entering through the first air inlet channels 12 to flow along the inner wall of the airflow channel 11.The airflows entering through the first air inlet channels 12 are used to form blocking airflows (as shown by arrows Q1 in FIG. 14, similarly hereinafter) between the inner wall of the atomization suction nozzle and the atomizing gas.

Further, the atomization suction nozzle further includes a second connection portion 32. The first airflow guide portion 31 is connected to the inner wall of the airflow channel 11 through the second connection portion 32.

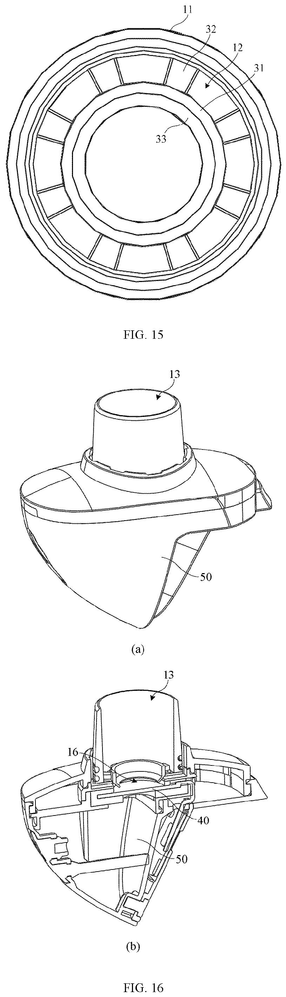

Specifically, referring to FIG. 15 together, a plurality of second connection portions 32 are arranged between the first airflow guide portion 31 and the inner wall of the airflow channel 11. The plurality of second connection portions 32 are spaced in a circumferential direction of the first airflow guide portion 31, and the first air inlet channel 12 is formed between adjacent second connection portions 32, that is, at least one first air inlet channel 12 is formed. In the way, a relative position of the first airflow guide portion 31 in the atomization suction nozzle is fixed, and formation of the first air inlet channels 12 between the first airflow guide portion 31 and the inner wall of the airflow channel 11 is ensured.

Alternatively, the first airflow guide portion 31 may be in an annular shape corresponding to an inner space of the atomization suction nozzle, and surrounds along a circumferential direction of the atomization suction nozzle.

In an embodiment, still referring to FIG. 14, the airflow guide member further includes a second airflow guide portion 33. The second airflow guide portion 33 is arranged on a side of the first airflow guide portion 31 away from the air outlet 13, that is, the first airflow guide portion 31 is closer to the air outlet 13 relative to the second airflow guide portion 33. The second airflow guide portion 33 is obliquely arranged in a direction away from the inner wall of the airflow channel 11 and the air outlet 13 to form the second air inlet channels 15, and airflows (as shown by arrows Q2 in FIG. 14, similarly hereinafter) entering through the second air inlet channels 15 are used to guide the atomizing gas to be output from the air outlet 13, thereby speeding up the discharge of the atomizing gas.

Specifically, the airflow entering through the second air inlet channel 15 flows to the first air inlet 16 along the second airflow guide portion 33 to be mixed with atomizing gas at the first air inlet 16, and then carries the atomizing gas to pass through the first air inlet 16 and to be output from the air outlet 13.

Alternatively, the second airflow guide portion 33 may be in an annular shape corresponding to an inner space of the atomization suction nozzle, and surrounds along a circumferential direction of the atomization suction nozzle.

It is to be noted that, in the exemplary embodiment, the airflow guide member is arranged at an end of the atomization suction nozzle away from the air outlet 13, so that the airflow guide member is as close as possible to the atomization core of the electronic atomization device after the atomization suction nozzle is assembled to the electronic atomization device. In this way, the airflow guided by the airflow guide member can drive the output of the atomizing gas near the atomization core to the most, and the problem of atomizing gas retention near the atomization core can be alleviated to the most, thereby alleviating the problem of atomizing gas condensation near the atomization core to the most.

Certainly, in other embodiments in the present disclosure, the airflow guide member and the second air inlet 18 communicated with the airflow guide member can be arranged at other positions in the axial direction of the atomization suction nozzle, and the problem of atomizing gas retention near the atomization core can also be alleviated, which is not limited herein.

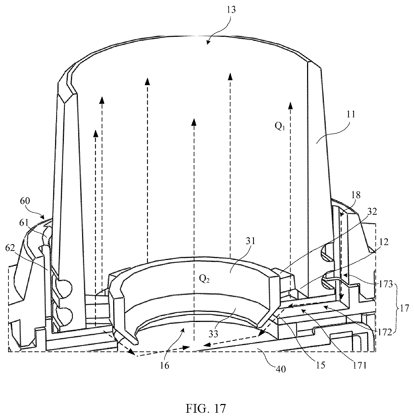

Referring to FIG. 16 and FIG. 17, FIG. 16 is a structural schematic view of a second embodiment of an atomizer according to the present disclosure, and FIG. 17 is a partial cross-sectional structural schematic view of a second embodiment of an atomizer according to the present disclosure.

An exemplary embodiment in which the air-curtain formation structure is an atomizer for the electronic atomization device is described below.

In the embodiment, the air-curtain formation structure is in a form of the atomizer. The atomizer provided in the embodiment is for electronic atomization devices such as an e-cigarette and a medical atomizer. FIG. 16 shows a case in which the air-curtain formation structure is for the medical atomizer, which is merely used for description and is not intended to limit an application environment of the air-curtain formation structure in the embodiment.

In the embodiment, referring to FIG. 16, the air-curtain formation structure includes an atomization suction nozzle, an atomization core 40, and a liquid storage cavity 50. The atomization suction nozzle defines a first air inlet 16 and an air outlet 13, atomizing gas flows into the atomization suction nozzle through the first air inlet 16 and is delivered to the air outlet 13 through the atomization suction nozzle, and then is output from the air outlet 13 for the user to suction. The atomization core 40 is arranged at a position of the first air inlet 16 of the atomization suction nozzle and is configured to atomize an aerosol generation substrate stored in the liquid storage cavity 50 to generate atomizing gas. Structures such as the atomization core 40, the liquid storage cavity 50 and the like constitute atomizing gas generation device of the air-curtain formation structure in the embodiment configured to generate atomizing gas.

For the case in which the air-curtain formation structure in the embodiment is for the medical atomizer, the atomization core 40 may be an ultrasonic atomization sheet, and the ultrasonic atomization sheet atomizes the aerosol generation substrate through high-frequency oscillation. The specific principle thereof falls within the understanding scope of a person skilled in the art, and details are not described herein again. Certainly, for the case in which the air-curtain formation structure is for other fields, the atomization core 40 may also generate atomizing gas in a manner of heating and atomizing the aerosol generation substrate, which is not limited herein.

Specifically, referring to FIG. 17, the atomization suction nozzle defines an airflow channel 11. The airflow channel 11 is configured to deliver atomizing gas. The atomization suction nozzle further includes first air inlet channels 12 communicated with the airflow channel 11, and are configured to introduce external airflows into the airflow channel 11, so that blocking airflows (as shown by arrows Q1 in FIG. 11, similarly hereinafter) are formed between the inner wall of the airflow channel 11 and the atomizing gas. The blocking airflows form an air curtain.

Further, the first air inlet 16 and the air outlet 13 are provided opposite to each other and are respectively communicated with the airflow channel 11. The first air inlet channels 12 are close to the inner wall of the airflow channel 11, and exits of the first air inlet channels 12 face the air outlet 13, to ensure that the airflows flowing into the airflow channel 11 through the first air inlet channels 12 can flow along the inner wall of the airflow channel 11 (i.e, an inner wall of the atomization suction nozzle), that is, the blocking airflows are formed to block the atomizing gas and the inner wall of the airflow channel 11, that is, block the atomizing gas and the inner wall of the atomization suction nozzle, so that the atomizing gas may be contacted with the inner wall of the atomization suction nozzle as little as possible, thereby alleviating the problem of atomizing gas condensation and reducing condensate generation.

Further, a flow direction of the blocking airflows is parallel to the inner wall of the airflow channel 11, that is, the flow direction of the blocking airflows is parallel to the inner wall of the atomization suction nozzle, to ensure a desirable effect of the blocking airflows for blocking the atomizing gas and the inner wall of the atomization suction nozzle.

In an embodiment, still referring to FIG. 17, the atomization suction nozzle further defines the second air inlet 18 different from the first air inlet 16, and the second air inlet 18 is configured to guide an external airflow to flow into the atomization suction nozzle. The atomization suction nozzle further includes an airflow guide member. The airflow guide member is communicated with the second air inlet 18 and is configured to guide the airflow entering through the second air inlet 18 to flow toward the first air inlet 16, and then carry atomizing gas flowing into the atomization suction nozzle through the first air inlet 16 and output the atomizing gas through the air outlet 13 of the atomization suction nozzle, so that the user can suction and the discharge of the atomizing gas can be accelerated.

In other words, the airflow guide member is configured to guide the airflow to flow toward the atomization core 40, to drive the atomizing gas near the atomization core 40 to be output from the air outlet 13, so that the problem of atomizing gas retention near the atomization core 40 can be effectively alleviated, thereby alleviating the problem of atomizing gas condensation near the atomization core 40.

Specifically, at least a part of the airflow guide member is obliquely arranged in a direction away from the inner wall and the air outlet 13 of the atomization suction nozzle, to guide the airflow entering through the second air inlet 18 to flow toward the first air inlet 16, that is, guide the airflow to flow toward the atomization core 40 to directly face a surface of the atomization core 40, to carry atomizing gas atomized by the atomization core 40 to flow into the atomization suction nozzle through the first air inlet 16 and to be output from the air outlet 13, and speed up the discharge of the atomizing gas, so that less atomizing gas is contacted with the inner wall of the atomization suction nozzle to some extent, thereby alleviating the problem of atomizing gas condensation, and reducing condensate generation.

In an embodiment, still referring to FIG. 17, the airflow guide member includes a first airflow guide portion 31. The first air inlet channels 12 are formed between the first airflow guide portion 31 and the inner wall of the airflow channel 11 (i.e, the inner wall of the atomization suction nozzle), and are configured to guide airflows entering through the first air inlet channels 12 to flow along the inner wall of the airflow channel 11. The airflows entering through the first air inlet channels 12 are used to form blocking airflows between the inner wall of the atomization suction nozzle and the atomizing gas.

Further, the atomization suction nozzle further includes a second connection portion 32. The first airflow guide portion 31 is connected to the inner wall of the airflow channel 11 through the second connection portion 32.

Specifically, a plurality of second connection portions 32 are arranged between the first airflow guide portion 31 and the inner wall of the airflow channel 11. The plurality of second connection portions 32 are spaced along a circumferential direction of the first airflow guide portion 31, and the first air inlet channel 12 is formed between adjacent second connection portions 32, that is, at least one first air inlet channel 12 is formed. In this way, a relative position of the first airflow guide portion 31 in the atomization suction nozzle is fixed, and formation of the first air inlet channels 12 between the first airflow guide portion 31 and the inner wall of the airflow channel 11 is ensured.

Alternatively, the first airflow guide portion 31 may be in an annular shape corresponding to an inner space of the atomization suction nozzle, and surrounds along a circumferential direction of the atomization suction nozzle. In an embodiment, still referring to FIG. 17, the airflow guide member further includes a second airflow guide portion 33. The second airflow guide portion 33 is arranged on a side of the first airflow guide portion 31 away from the air outlet 13, that is, the first airflow guide portion 31 is closer to the air outlet 13 relative to the second airflow guide portion 33. The second airflow guide portion 33 is obliquely arranged in a direction away from the inner wall of the airflow channel 11 and the air outlet 13 to form the second air inlet channels 15, and airflows entering through the second air inlet channels 15 are used to guide the atomizing gas to be output from the air outlet 13, thereby speeding up the discharge of the atomizing gas.

Specifically, the airflow entering through the second air inlet channel 15 flows to the first air inlet 16 along the second airflow guide portion 33 to be mixed with atomizing gas at the first air inlet 16, and then carries the atomizing gas to pass through the first air inlet 16 and to be output from the air outlet 13.

Alternatively, the second airflow guide portion 33 may be in an annular shape corresponding to an inner space of the atomization suction nozzle, and surrounds along a circumferential direction of the atomization suction nozzle.

In an embodiment, still referring to FIG. 17, the air-curtain formation structure further defines a converging channel 17, one end of the converging channel 17 is an air inlet, that is, a second air inlet 18, and the other end of the converging channel 17 is a diverging opening 171, and the diverging opening 171 is respectively communicated with the first air inlet channels 12 and the second air inlet channels 15.

Specifically, the converging channel 17 has a first channel section 172 and a second channel section 173 communicated with each other, an end opening of the first channel section 172 away from the second channel section 173 is the diverging opening 171, and an end opening of the second channel section 173 away from the first channel section 172 is the air inlet, that is, the second air inlet 18. An extending direction of the first channel section 172 is different from an extending direction of the second channel section 173.

FIG. 17 shows that the extending direction of the first channel section 172 is a horizontal direction, the extending direction of the second channel section 173 is a vertical direction, and the second channel section 173 extends toward the air outlet 13. When the user suctions, an external airflow flows into the second channel section 173 through the second air inlet 18 and is delivered into the first channel section 172, and then the airflow passes through the diverging opening 171 and flows into the airflow channel 11 in the atomization suction nozzle respectively through the first air inlet channels 12 and the second air inlet channels 15. Flow conditions of airflows are shown by dashed arrows in FIG. 17.

Further, the air-curtain formation structure includes a mounting portion 60. The mounting portion 60 includes a mounting protrusion 61 and a vent groove 62. The mounting protrusion 61 is configured to fix the atomization suction nozzle. After the atomization suction nozzle is fixed to the mounting portion 60, the first channel section 172 is formed between the atomization suction nozzle and the mounting portion 60, and to be specific, the first channel section 172 is formed between the atomization suction nozzle and the bottom of the mounting portion 60. In addition, the second channel section 173 is formed between the vent groove 62 and the atomization suction nozzle.

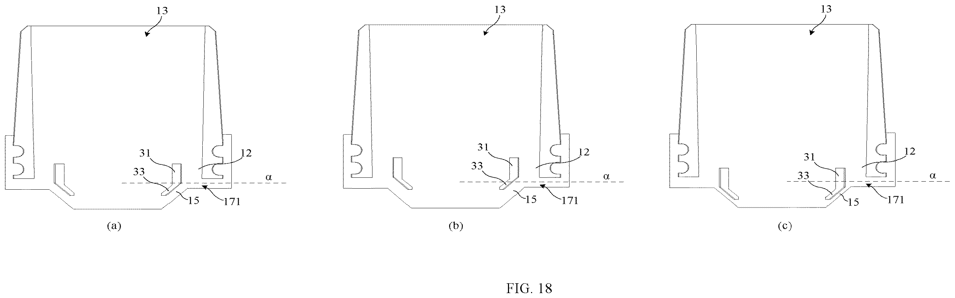

Referring to FIG. 18, FIG. 18 is a structural schematic view of a relative position relationship between a center line of a diverging opening and a joint of a first airflow guide portion and a second airflow guide portion of an atomizer according to the present disclosure. Airflow in the first air inlet channel 12 and the second air inlet channel 15 in the exemplary embodiment is described below.

**In** the exemplary embodiment, the airflow entering through the first air inlet channel 12 forms the blocking airflow between the inner wall of the atomization suction nozzle and the atomizing gas, so that the atomizing gas is contacted with the inner wall of the atomization suction nozzle as little as possible, thereby alleviating the problem of atomizing gas condensation, and reducing condensate generation. The airflow entering through the second air inlet channel 15 guides the atomizing gas to be output from the air outlet 13, to speed up the discharge of the atomizing gas, thereby effectively alleviating the problem of atomizing gas condensation in a cavity surrounded by the airflow guide member.

Since the air pressure difference caused by user suctioning is fixed, a total amount of the airflows flowing into the first air inlet channel 12 and the second air inlet channel 15 is fixed. Therefore, in the exemplary embodiment, the amount of the airflows flowing into the first air inlet channel 12 and the second air inlet channel 15 is appropriately allocated, to alleviate the problem of atomizing gas condensation on the inner wall of the atomization suction nozzle and in the cavity surrounded by the airflow guide member.

In an embodiment, a center line α of the diverging opening 171 the center line α of the diverging opening 171 being defined as being perpendicular to a central axis of the diverging opening 171, similarly hereinafter) extends through the joint of the first airflow guide portion 31 and the second airflow guide portion 33, as shown in FIG. 18a. In this way, the airflow entering through the first air inlet channel 12 is sufficient to form the blocking airflow between the inner wall of the atomization suction nozzle and the atomizing gas, thereby reducing the adhesion degree of the atomizing gas on the inner wall of the atomization suction nozzle. In addition, the airflow entering through the second air inlet channel 15 is sufficient to quickly carry and discharge the atomizing gas, thereby reducing the adhesion degree of the atomizing gas in the cavity surrounded by the airflow guide member.

In an alternative embodiment, the center line α of the diverging opening 171 is away from the air outlet 13 relative to the joint of the first airflow guide portion 31 and the second airflow guide portion 33, as shown in FIG. 18b. In this way, the amount of the airflow entering through the second air inlet channel 15 is significantly increased, thereby further speeding up carrying and discharging the atomizing gas, further reducing the adhesion degree of the atomizing gas in the cavity surrounded by the airflow guide member, and alleviating the problem of atomizing gas condensation in the cavity surrounded by the airflow guide member.

In another alternative embodiment, the center line α of the diverging opening 171 is close to the air outlet 13 relative to the joint of the first airflow guide portion 31 and the second airflow guide portion 33, as shown in FIG. 18c. In this way, the amount of the airflow entering through the first air inlet channel 12 is significantly increased, thereby further increasing an amount of the blocking airflows between the inner wall of the atomization suction nozzle and the atomizing gas, and further reducing the adhesion degree of the atomizing gas on the inner wall of the atomization suction nozzle, and alleviating the problem of atomizing gas condensation on the inner wall of the atomization suction nozzle.

It is to be noted that, a size relationship between the cross-sectional area of the first air inlet channel 12 and the cross-sectional area of the second air inlet channel 15 is the same as an airflow amount relationship between the airflow of the first air inlet channel 12 and the airflow of the second air inlet channel 15. That is to say, the cross-sectional area of the first air inlet channel 12 being greater than the cross-sectional area of the second air inlet channel 15 indicates that the airflow amount of the first air inlet channel 12 being greater than the airflow amount of the second air inlet channel 15, or otherwise, the opposite.

In view of this, in the exemplary embodiment, the cross-sectional area of the second air inlet channel 15 can be adjusted by adjusting a degree of inclination of the second airflow guide portion 33 of the airflow guide member, thereby adjusting the size relationship between the cross-sectional area of the first air inlet channel 12 and that of the second air inlet channel 15, and adjusting the airflow amount of the first air inlet channel 12 and the second air inlet channel 15.

Specifically, the second airflow guide portion 33 being more inclined in a direction away from the air outlet 13 indicates a smaller cross-sectional area of the second air inlet channel 15, and a smaller airflow amount of the second air inlet channel 15 and a larger airflow amount of the first air inlet channel 12, or otherwise, the opposite.

It is to be noted that, in the foregoing manner, the adhesion degrees of the atomizing gas on the inner wall of the atomization suction nozzle and the atomizing gas in the cavity surrounded by the airflow guide member are less than 3%. It can be seen that, based on the design of the first air inlet channel 12 and the second air inlet channel 15 in the exemplary embodiment, the adhesion degree of the atomizing gas can be effectively reduced, thereby alleviating the problem of atomizing gas condensation.

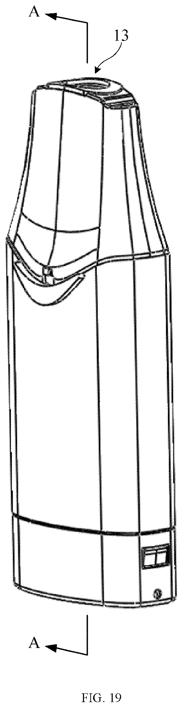

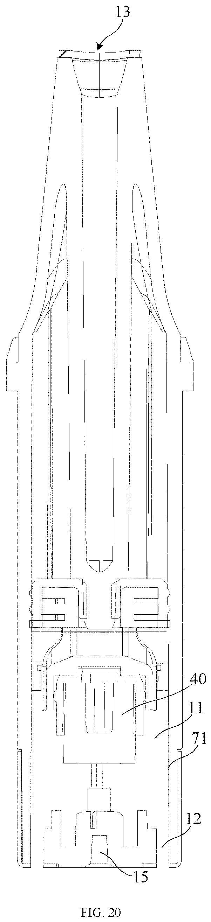

Referring to FIG. 19 and FIG. 20, FIG. 19 is a structural schematic view of a third embodiment of an atomizer according to the present disclosure, and FIG. 20 is a cross-sectional structural schematic view of a third embodiment of an atomizer in a direction A-A according to the present disclosure.

An exemplary embodiment in which the air-curtain formation structure is an atomizer for the electronic atomization device is described below.

In the embodiment, the air-curtain formation structure is in a form of the atomizer. The atomizer provided in the embodiment is for electronic atomization devices such as an e-cigarette and a medical atomizer. FIG. 19 shows a case in which the air-curtain formation structure is for the e-cigarette, which is merely used for description and is not intended to limit an application environment of the air-curtain formation structure in the embodiment.

Specifically, the atomizer defines an airflow channel 11. The airflow channel 11 is configured to deliver atomizing gas. The atomizer further defines a first air inlet channel 12 communicated with the airflow channel 11, and the first air inlet channel 12 is configured to introduce an external airflow into the airflow channel 11, so that a blocking airflow is formed between an inner wall of the airflow channel 11 and the atomizing gas. The blocking airflows form an air curtain.

Further, the atomizer further defines an air outlet 13 communicated with the airflow channel 11, the first air inlet channel 12 is close to the inner wall of the airflow channel 11, and an exit of the first air inlet channel 12 faces the air outlet 13, to ensure that the airflow flowing into the airflow channel 11 through the first air inlet channel 12 can flow along the inner wall of the airflow channel 11 (i.e, an inner wall of the atomizer), that is, the blocking airflow are formed to block the atomizing gas and the inner wall of the airflow channel 11, that is, block the atomizing gas and the inner wall of the atomizer, so that the atomizing gas may be contacted with the inner wall of the atomizer as little as possible, thereby alleviating the problem of atomizing gas condensation and reducing condensate generation.

In an embodiment, still referring to FIG. 20, the atomizer further defines an atomization cavity 71. An atomization core 40 is arranged in the atomization cavity 71 and is configured to atomize an aerosol generation substrate to generate atomizing gas. The airflow channel 11 is provided in the atomization cavity 71, that is, a space used for accommodating the atomization cavity 71 is the airflow channel 11. A first air inlet channel 12 is provided on the bottom of the atomization cavity 71 close to an inner wall of the atomization cavity 71, so that an airflow flowing into the atomization cavity 71 through the first air inlet channel 12 during user suctioning may flow along the inner wall of the atomization cavity 71, thereby forming a blocking airflow between the inner wall of the atomization cavity 71 and the atomizing gas.

Further, the atomizer further defines a second air inlet channel 15, an airflow entering through the second air inlet channel 15 has ability of guiding the atomizing gas to be output from the air outlet 13, thereby speeding up the discharge of the atomizing gas,reducing contact between atomizing gas and the inner wall of the atomization cavity 71 to some extent, and similarly alleviating the problem of atomizing gas condensation. Specifically, the second air inlet channel 15 is provided on the bottom of the atomization cavity 71, and the first air inlet channel 12 is close to an edge of the bottom of the atomization cavity 71 relative to the second air inlet channel 15.

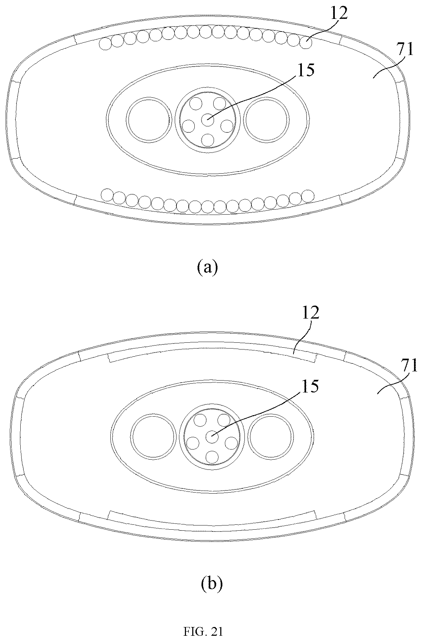

Furthermore, referring to FIG. 21, the first air inlet channels 12 are respectively provided on two opposite sides of the second air inlet channels 15. Based on the foregoing manner, a quantity of the first air inlet channels 12 can be increased, thereby further reducing contact between the atomizing gas and the inner wall of the atomization cavity 71, and further alleviating the problem of atomizing gas condensation. In addition, the first air inlet channels 12 are provided on the two opposite sides of the second air inlet channels 15 as symmetrically as possible, thereby optimizing the distribution of the blocking airflows in the atomization cavity 71, and improving the effect of alleviating the problem of atomizing gas condensation.

In an embodiment, the first air inlet channels 12 may be in a through-hole form, as shown in FIG. 21a. A plurality of first air inlet channels 12 are spaced on the bottom of the atomization cavity 71 close to the inner wall of the atomization cavity 71, and airflows flowing into the atomization cavity 71 through the first air inlet channels 12 in the through-hole form may form blocking airflows. Specifically, the plurality of first air inlet channels 12 are spaced along an edge on the bottom of the atomization cavity 71, and the plurality of first air inlet channels 12 are spaced on the two opposite sides of the second air inlet channels 15.

Alternatively, the hole diameter of the first air inlet channel 12 in the through-hole form may be 0.3 mm, 0.4 mm, or the like, which is not limited herein.

In an alternative embodiment, a cross section of the first air inlet channel 12 is strip-shaped, that is, the first air inlet channel 12 is a strip-shaped narrow gap, as shown in FIG. 21b. The first air inlet channels 12 in the narrow-gap form extend along the edge on the bottom of the atomization cavity 71, and airflows flowing into the atomization cavity 71 through the first air inlet channels 12 in the narrow-gap form may form the blocking airflows. Further, the first air inlet channels 12 in the narrow-gap form are respectively provided on the two opposite sides of the second air inlet channels 15.

Alternatively, a width of the first air inlet channel 12 in the narrow-gap form may be 0.3 mm, 0.4 mm, or the like, which is not limited herein.

It is to be noted that, distribution of the blocking airflows formed by the airflows entering through the first air inlet channel 12 in the narrow-gap form is better than distribution of the blocking airflows formed by the airflows entering through the first air inlet channel 12 in the through-hole form, and distribution of the blocking airflows formed by the airflows entering through the first air inlet channel 12 of a width of 0.4 mm is better than distribution of the blocking airflows formed by the airflows entering through the first air inlet channel 12 of a width of 0.3 mm. In addition, an entire flow direction of airflows inside the atomization cavity 71 is more ordered because of function of the blocking airflows, so that a vortex flow is unlikely to be formed.

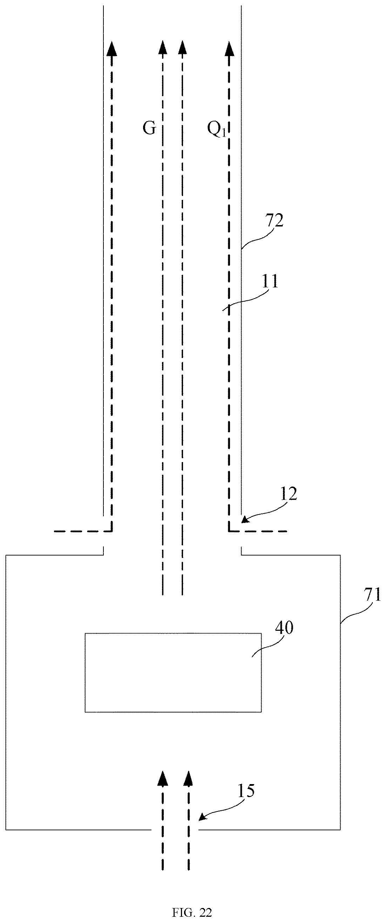

Referring to FIG. 22, FIG. 22 is a structural schematic view of a fourth embodiment of an atomizer according to the present disclosure.

An exemplary embodiment in which the air-curtain formation structure is an atomizer for the electronic atomization device is described below.

In the embodiment, the air-curtain formation structure is in a form of the atomizer. The atomizer provided in the embodiment is for electronic atomization devices such as an e-cigarette and a medical atomizer. FIG. 22 shows a case in which the air-curtain formation structure is for the e-cigarette, which is merely used for description and is not intended to limit an application environment of the air-curtain formation structure in the embodiment.

Specifically, the atomizer defines an airflow channel 11. The airflow channel 11 is configured to deliver atomizing gas. The atomizer further defines a first air inlet channel 12 communicated with the airflow channel 11, and the first air inlet channel 12 is configured to introduce an external airflow into the airflow channel 11, thereby forming a blocking airflow between an inner wall of the airflow channel 11 and the atomizing gas. The blocking airflows form an air curtain.

The atomizer further defines an air outlet channel 72, the airflow channel 11 is provided in the air outlet channel 72, and the first air inlet channels 12 are provided on a side wall of the air outlet channel 72. When the user suctions, external airflows flow into the air outlet channel 72 through the first air inlet channels 12 on the side wall of the air outlet channel 72 and then flow along an inner wall of the air outlet channel 72, thereby forming blocking airflows between the inner wall of the air outlet channel 72 and the atomizing gas, effectively reducing contact between high-temperature atomizing gas in the air outlet channel 72 and the inner wall of the low-temperature air outlet channel 72, and reducing atomizing gas condensation. As shown in FIG. 22, blocking airflows Q1 are arranged between the inner wall of the air outlet channel 72 and atomizing gas G, to block the inner wall of the air outlet channel 72 and the atomizing gas G.

Further, the atomizer further defines an atomization cavity 71. An atomization core 40 is arranged in the atomization cavity 71 and is configured to atomize an aerosol generation substrate to generate atomizing gas. The atomization cavity 71 is communicated with the air outlet channel 72. In addition, a second air inlet channel 15 is provided in the atomization cavity 71. When the user suctions, external airflows flow into the atomization cavity 71 through the second air inlet channels 15, to carry the atomizing gas in the atomization cavity 71 to be discharged through the air outlet channel 72, thereby speeding up the discharge of the atomizing gas, reducing contact between the atomizing gas and the inner wall of the atomization cavity 71, reducing contact between the atomizing gas and the inner wall of the air outlet channel 72 to some extent, and alleviating the problem of atomizing gas condensation.

The first air inlet channels 12 are provided on a part of the air outlet channel 72 close to the atomization cavity 71, as shown in FIG. 22, thereby avoiding the atomizing gas condensation of the air outlet channel 72 arranged between the first air inlet channels 12 and the atomization cavity 71 as much as possible, and further alleviating the problem of atomizing gas condensation.

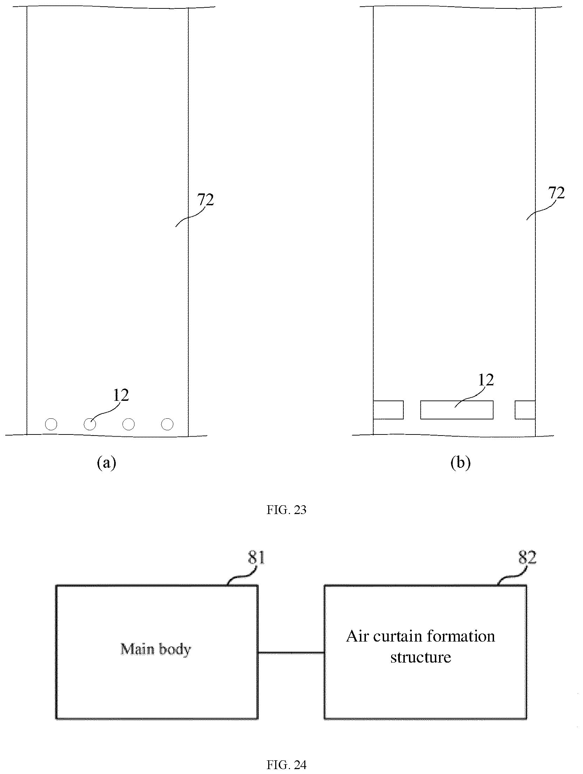

Further, referring to FIG. 23, the atomizer defines a plurality of first air inlet channels 12, and the plurality of first air inlet channels 12 are sequentially spaced apart from each other along a circumferential direction of the air outlet channel 72. Furthermore, the plurality of first air inlet channels 12 are evenly spaced along the circumferential direction of the air outlet channel 72, thereby evenly flowing airflows into the side wall of the air outlet channel 72, and further forming blocking airflows in the air-curtain form that are well distributed in the air outlet channel 72.

Alternatively, the first air inlet channels 12 are preferably circular holes as shown in FIG. 23a or elongated holes as shown in FIG. 23b. In addition, a diameter of the first air inlet channel 12 in the circular-hole form may be 0.3 mm, 0.4 mm, or the like, and a width of the first air inlet channel 12 in the strip-shaped form may be 0.3 mm, 0.4 mm, or the like, which are not limited herein.

Referring to table in the following, the table shows an accumulation amount of condensate in a conventional air outlet channel and the air outlet channel 72 in the exemplary embodiment when the user suctions for different times.

| | Accumulation amount of condensate/mg | | |
|---|---|---|---|
| | 10/times | 30/times | 50/times |
| Conventional air outlet channel | 1.1 | 2.2 | 4.8 |
| Air outlet channel in the exemplary embodiment | 0.8 | 1.9 | 4.7 |

Based on the above, an air-curtain formation structure for an electronic atomization device is provided in the present disclosure, and the air-curtain formation structure defines an airflow channel configured to deliver atomizing gas. The airflow channel has a first air inlet channel, and the first air inlet channel is configured to introduce an external airflow into the airflow channel, so that a blocking airflow is formed between an inner wall of the airflow channel and the atomizing gas. In the present disclosure, the blocking airflow is used to block the inner wall of the airflow channel and the atomizing gas, so that the atomizing gas is contacted with the inner wall of the airflow channel as little as possible, the problem of atomizing gas condensation can be alleviated, and less condensate is generated, thereby improving the user experience, reducing drug loss, and reducing the risk of condensate leakage.

Referring to FIG. 24, FIG. 24 is a structural schematic view of an embodiment of an electronic atomization device according to the present disclosure.

In the embodiment, the electronic atomization device may be an e-cigarette or a medical atomization electronic device, and includes a main body 81 and an air-curtain formation structure 82, the main body 81 is connected to the air-curtain formation structure 82, and the air-curtain formation structure 82 defines an airflow channel configured to deliver atomizing gas. The air-curtain formation structure 82 further defines a first air inlet channel communicated with the airflow channel, and the first air inlet channel is configured to introduce an external airflow into the airflow channel, so that a blocking airflow is formed between an inner wall of the airflow channel and the atomizing gas.

The air-curtain formation structure 82 is described in detail in the foregoing embodiments, and details are not described herein again.

It is to be noted that, the main body 81 is defined as a set of elements of the electronic atomization device other than the air-curtain formation structure 82. Specifically, when the air-curtain formation structure 82 is an atomization suction nozzle for the electronic atomization device, the main body 81 includes a main unit (including a power supply and circuit parts of the electronic atomization device) of the electronic atomization device and other elements (including an atomization core, and the like) of the atomizer other than the atomization suction nozzle. In addition, when the air-curtain formation structure 82 is the atomizer for the electronic atomization device, the main body 81 includes the main unit of the electronic atomization device.

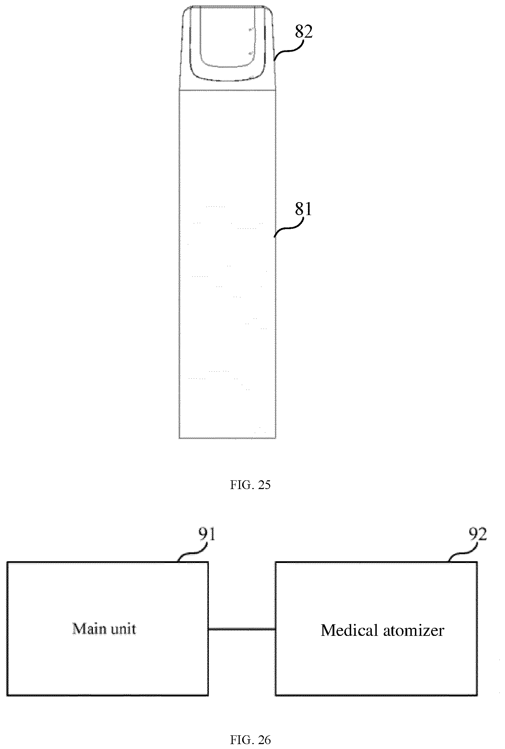

For example, FIG. 25 shows an overall configuration of the main body 81 and the air-curtain formation structure 82 after assembly, that is, the electronic atomization device.

Referring to FIG. 26, FIG. 26 is a structural schematic view of an embodiment of a medical atomization electronic device according to the present disclosure.

In the embodiment, the medical atomization electronic device is for the field of medical atomization and includes a main unit 91 (including a power supply and circuit parts of the medical atomization electronic device) and a medical atomizer 92 connected to the main unit 91. The medical atomizer 92 includes an atomization suction nozzle, and the atomization suction nozzle defines a first air inlet, a second air inlet, and an air outlet. The medical atomizer 92 further defines a liquid storage cavity, and the liquid storage cavity is configured to store an aerosol generation substrate. The medical atomizer 92 further includes an atomization core, and the atomization core is arranged in the first air inlet and is configured to atomize the aerosol generation substrate to generate atomizing gas. The medical atomizer 92 further includes an airflow guide member, the airflow guide member is arranged in the atomization suction nozzle and is communicated with the second air inlet, and the airflow guide member is configured to guide an airflow entering through the second air inlet to flow toward the atomization core, to carry the atomizing gas to be output from the air outlet.

The medical atomizer 92 is described in detail in the foregoing embodiments, and details are not described herein again.

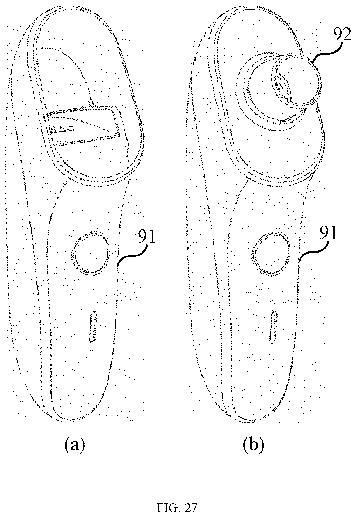

For example, FIG. 27a shows an exemplary embodiment of the main unit 91, and FIG. 27b shows an overall configuration of the main unit 91 and the medical atomizer 92 after assembly, that is, the medical atomization electronic device.

In the present disclosure, unless otherwise explicitly specified or defined, the terms such as "connect", "connection", and "stack" should be understood in a broad sense. For example, the connection may be a fixed connection, a detachable connection, or an integral connection; or the connection may be a direct connection, an indirect connection through an intermediate medium, internal communication between two elements, or an interaction relationship between two elements. A person of ordinary skill in the art may understand the specific meanings of the foregoing terms in the present disclosure according to specific situations.

Finally, it is to noted that the foregoing embodiments are merely used for describing technical solutions of the present disclosure, but are not intended to limit the present disclosure. Although the present disclosure is described in detail with reference to the foregoing embodiments, a person of ordinary skill in the art is to understand that, modifications may still be made to the technical solutions in the foregoing embodiments, or equivalent replacements may be made to some or all of the technical features; and these modifications or replacements will not cause the essence of corresponding technical solutions to depart from the scope of the technical solutions in the embodiments of the present disclosure.

## Claims

1. An air-curtain formation structure (82) for an electronic atomization device, having:
an airflow channel (11), configured to deliver atomizing gas (G);
a first air inlet channel (12), communicated with the airflow channel (11) and configured to introduce an external airflow into the airflow channel (11), so that a blocking airflow (Q1) is formed between the inner wall of the airflow channel (11) and the atomizing gas (G), and a flow direction of the blocking airflow (Q1) is parallel to the inner wall of the airflow channel (11); and
an air outlet (13), communicated with the airflow channel (11);
wherein the first air inlet channel (12) is close to the inner wall of the airflow channel (11), and an exit of the first air inlet channel (12) faces the air outlet (13),
wherein the air-curtain formation structure (82) further comprises a first airflow guide portion (31),
the first air inlet channel (12) is formed between the first airflow guide portion (31) and the inner wall of the airflow channel (11), and
the first airflow guide portion (31) is configured to guide the airflow introduced through the first air inlet channel (12) to flow along the inner wall of the airflow channel (11), to form the blocking airflow (Q1),
wherein the air-curtain formation structure (82) further comprises a second airflow guide portion (33),
the second airflow guide portion (33) is obliquely arranged in a direction away from the inner wall of the airflow channel (11) and the air outlet (13) to form a second air inlet channel (15), and
an airflow entering through the second air inlet channel (15) is used to guide the atomizing gas (G) to be output from the air outlet (13).

2. The air-curtain formation structure (82) as claimed in claim 1, wherein
the air-curtain formation structure (82) is an atomization suction nozzle for the electronic atomization device; and
the atomization suction nozzle comprises an airway body (21) and a tube body (22), the airflow channel (11) is provided in the airway body (21) and the tube body (22), an end of the tube body (22) away from the airway body (21) is the air outlet (13), and the first air inlet channel (12) is provided at a position of the airway body (21) close to the inner wall of the tube body (22), so that the blocking airflow (Q1) is formed between the inner wall of the tube body (22) and the atomizing gas (G).

3. The air-curtain formation structure (82) as claimed in claim 2, wherein
the airway body (21) comprises a first airway portion (212), a second airway portion (213), and a wall portion (211); and the airflow channel (11) further has an entrance channel (111) and an air guide channel (112) communicated with each other, the entrance channel (111) is provided in the first airway portion (212), the air guide channel (112) is provided in the tube body (22), the second airway portion (213) is sleeved on a periphery of the tube body (22), and the wall portion (211) is connected to the first airway portion (212) and the second airway portion (213) and covers the air guide channel (112); and
wherein the first air inlet channel (12) is provided on the wall portion (211).

4. The air-curtain formation structure (82) as claimed in claim 3, wherein the airflow channel (11) has a plurality of first air inlet channels (12) spaced apart from each other along a circumferential direction of the wall portion (211); or
wherein the first airway portion (212) further comprises a vent portion (214) and a first connection portion (215), and the vent portion (214) is connected to the wall portion (211) through the first connection portion (215); and
a cross-sectional area of the first connection portion (215) is less than a cross-sectional area of the vent portion (214), so that a clamping opening (216) is formed at a position of the first connection portion (215) is formed, and the clamping opening (216) is configured to clamp an atomizing gas generation device of the electronic atomization device.

5. The air-curtain formation structure (82) as claimed in claim 4, wherein a second air inlet channel (15) is provided in the vent portion (214), and an airflow (Q2) entering through the second air inlet channel (15) has ability of guiding the atomizing gas (G) to be output from the air outlet (13).

6. The air-curtain formation structure (82) as claimed in claim 5, wherein the second air inlet channel (15) has an air inlet portion (151) and an air guide portion (152), the air inlet portion (151) is configured to introduce the external airflow (Q2) into the air guide portion (152), the air inlet portion (151) is communicated with the airflow channel (11) through the air guide portion (152), and an extending direction of the air guide portion (152) is parallel to an extending direction of the entrance channel (111); or
wherein a comprehensive airway (14) is formed at a position of the clamping opening (216), and the comprehensive airway (14) is respectively communicated with the first air inlet channel (12) and the second air inlet channel (15); or
wherein the first air inlet channel (12) is provided corresponding to the second air inlet channel (15); or the first air inlet channel (12) and the second air inlet channel (15) are staggered; or
wherein a cross-sectional area of the first air inlet channel (12) is greater than a cross-sectional area of the second air inlet channel (15); and/or a quantity of the first air inlet channels (12) is greater than a quantity of the second air inlet channels (15).

7. The air-curtain formation structure (82) as claimed in claim 1, wherein the air-curtain formation structure (82) further comprises a second connection portion (32), and the first airflow guide portion (31) is connected to the inner wall of the airflow channel (11) through the second connection portion (32); or
wherein a plurality of second connection portions (32) are arranged between the first airflow guide portion (31) and the inner wall of the airflow channel (11), the plurality of second connection portions (32) are sequentially spaced along a circumferential direction of the first airflow guide portion (31), and the first air inlet channel (12) is formed between adjacent second connection portions (32).

8. The air-curtain formation structure (82) as claimed in claim 1, wherein an angle between a flow direction of the airflow entering through the second air inlet channel (15) and a preset direction ranges from 30° to 45°, and the preset direction is parallel to a flow direction of the blocking airflow (Q1).

9. The air-curtain formation structure (82) as claimed in claim 1, wherein the air-curtain formation structure (82) further comprises a third connection portion (34), and the second airflow guide portion (33) is connected to the first airflow guide portion (31) through the third connection portion (34); or
wherein a plurality of third connection portions (34) are arranged between the second airflow guide portion (33) and the first airflow guide portion (31), the plurality of third connection portions (34) are sequentially spaced along a circumferential direction of the second airflow guide portion (33), and the second air inlet channel (15) is formed between adjacent third connection portions (34).

10. The air-curtain formation structure (82) as claimed in claim 1, wherein the air-curtain formation structure (82) further comprises a second airflow guide portion (33) arranged on one side of the first airflow guide portion (31) away from the air outlet (13), and the second airflow guide portion (33) is obliquely arranged in a direction away from the inner wall of the airflow channel (11) and the air outlet (13) to form a second air inlet channel (15), and an airflow entering through the second air inlet channel (15) has ability of guiding the atomizing gas (G) to be output from the air outlet (13).

11. The air-curtain formation structure (82) as claimed in claim 10, wherein the air-curtain formation structure (82) is an atomizer for the electronic atomization device, the atomizer comprises an atomization suction nozzle and an atomizing gas generation device, the second airflow guide portion (33) is arranged in the atomization suction nozzle, and the second air inlet channel (15) is formed between the second airflow guide portion (33) and the atomizing gas generation device.

12. The air-curtain formation structure (82) as claimed in claim 11, wherein the air-curtain formation structure (82) further defines a converging channel (17), one end of the converging channel (17) is an air inlet, the other end of the converging channel (17) is a diverging opening (171), and the diverging opening (171) is respectively communicated with the first air inlet channel (12) and the second air inlet channel (15).

13. The air-curtain formation structure (82) as claimed in claim 12, wherein a center line (α) of the diverging opening (171) extends through a joint of the first airflow guide portion (31) and the second airflow guide portion (33); or
wherein a center line (α) of the diverging opening (171) is away from the air outlet (13) relative to a joint of the first airflow guide portion (31) and the second airflow guide portion (33); or
wherein a center line (α) of the diverging opening (171) is close to the air outlet (13) relative to a joint of the first airflow guide portion (31) and the second airflow guide portion (33); or
wherein the converging channel (17) has a first channel section (172) and a second channel section (173) that are communicated with each other, an end opening of the first channel section (172) away from the second channel section (173) is the diverging opening (171), and an end opening of the second channel section (173) away from the first channel section (172) is the air inlet; and an extending direction of the first channel section (172) is different from that of the second channel section (173).

14. The air-curtain formation structure (82) as claimed in claim 11, wherein the atomizing gas generation device comprises a mounting portion (60), the mounting portion (60) is provided with a mounting protrusion (61) and a vent groove (62), the mounting protrusion (61) is configured to fix the atomization suction nozzle, the first channel section (172) is formed between the atomization suction nozzle and the mounting portion (60), and the second channel section (173) is formed between the vent groove (62) and the atomization suction nozzle; or
wherein a periphery of the atomization suction nozzle is provided with limiting grooves (35) surrounding along a circumferential direction thereof, and the limiting grooves (35) are configured to place elastic rings, so that the atomization suction nozzle is fixed to the atomizing gas generation device.

15. The air-curtain formation structure (82) as claimed in claim 1, wherein
the air-curtain formation structure (82) is an atomizer for the electronic atomization device, and the atomizer defines an atomization cavity (71); and
the airflow channel (11) is provided in the atomization cavity (71), and the first air inlet channel (12) is provided at a position on a bottom of the atomization cavity (71) close to the inner wall of the atomization cavity (71), so that the blocking airflow (Q1) is formed between the inner wall of the atomization cavity (71) and the atomizing gas (G).

16. The air-curtain formation structure (82) as claimed in claim 15, wherein the atomizer further defines a second air inlet channel (15), an airflow entering through the second air inlet channel (15) has ability of guiding the atomizing gas (G) to be output from the air outlet (13), the second air inlet channel (15) is provided on the bottom of the atomization cavity (71), and the first air inlet channel (12) is close to an edge of the bottom of the atomization cavity (71) relative to the second air inlet channel (15).

17. The air-curtain formation structure (82) as claimed in claim 16, wherein the first air inlet channel (12) is respectively provided on two opposite sides of the second air inlet channel (15).

18. The air-curtain formation structure (82) as claimed in claim 15, wherein
the air-curtain formation structure (82) defines a plurality of first air inlet channels (12), and the plurality of first air inlet channels (12) are spaced apart from each other; and/or a cross section of the first air inlet channel (12) is strip-shaped.

19. The air-curtain formation structure (82) as claimed in claim 1, wherein
the air-curtain formation structure (82) is an atomizer for the electronic atomization device, and the atomizer defines an air outlet channel (72); and
the airflow channel (11) is provided in the air outlet channel (72), and the first air inlet channel (12) is provided on a side wall of the air outlet channel (72), so that the blocking airflow (Q1) is formed between the inner wall of the air outlet channel (72) and the atomizing gas (G).

20. The air-curtain formation structure (82) as claimed in claim 19, wherein the atomizer further defines an atomization cavity (71), the atomization cavity (71) is communicated with the air outlet channel (72), and the first air inlet channel (12) is provided on a part of the air outlet channel (72) close to the atomization cavity (71); or
wherein the atomizer defines a plurality of first air inlet channels (12) sequentially spaced apart from each other in a circumferential direction of the air outlet channel (72); or
wherein the first air inlet channel (12) is a circular hole or an elongated hole.

21. An electronic atomization device, wherein the electronic atomization device comprises a main body (81) and an air-curtain formation structure (82), the main body (81) is connected to the air-curtain formation structure (82), and the air-curtain formation structure (82) is as claimed in any one of claims 1-20.

## Patentansprüche

1. Eine Struktur (82) zur Bildung eines Luftvorhangs für eine elektronische Zerstäubungsvorrichtung, Struktur, die Folgendes umfasst:
Einen Luftstromkanal (11), der für die Zuführung von Zerstäubungsgas (G) konfiguriert ist;
ein erster Lufteintrittskanal (12), der mit dem Luftstromkanal (11) in Verbindung steht und konfiguriert ist, um einen externen Luftstrom in den Luftstromkanal (11) einzuleiten, so dass ein blockierender Luftstrom (Q1) zwischen der Innenwand des Luftstromkanals (11) und dem Zerstäubungsgas (G) gebildet wird, und eine Stromrichung des blockierenden Lufstroms (Q1) parallel zur Innenwand des Luftstromkanals (11) verläuft; und
ein Luftaustritt (13), der mit dem Luftstromkanal (11) kommuniziert;
wobei
der erste Lufteintrittskanal (12) nahe der Innenwand des Luftstromkanals (11) liegt und ein Austritt des ersten Lufteintrittskanals (12) zum Luftaustritt (13) gerichtet ist,
wobei die Strucktur (82) für die Bildung eines Luftvorhangs weiter einen ersten Luftstromführungsteil (31) umfasst,
der erste Lufteintrittskanal (12) zwischen dem Luftstromführungsteil (31) und der Innenwand des Luftstromkanals (11) gebildet ist, und
der erste Luftstromführungsteil (31) konfiguriert ist, um den durch den ersten Lufteintrittskanal (12) eingeführtenLuftstrom zu führen, damit er längs der Innenwand des Luftstromkanals (11) fliesst, um den blckierenden Luftstrom (Q1) zu bilden,
wobei die Struktur (82) für die Bildung des Luftvorhangs weiter einen zweiten Luftstromführungsteil (33) umfasst,
der zweite Luftstromführungsteil (33) schräg weggerichtet von der Innenwand des Luftstromkanals (11) und dem Luftaustritt (13) angeordnet ist zwecks Bildung eines zweiten Lufteintritttkanals (15), und
wobei der durch den zweiten Lufteintrittskanal (15) eintretende Luftstrom benutzt wird, um das Zerstäubungsgas (G) zwecks Abgabe durch den Luftaustritt (13) zu führen.

2. Die Struktur (82) zur Bildung eines Luftvorhangs (82) gemäss Anspruch 1,
wobei die Struktur (82) zur Bildung eines Luftvorhangs eine Zerstäubungssaugdüse für die elektronische Zerstäubungsvorrichtung ist; und
die Zerstäubungssaugdüse einen Luftbahnkörper (21) und einen Rohrkörper (22) umfasst, und der Luftstromkanal (11) im Luftbahnkörper (21) und Rohrkörper (22) vorgesehen ist, ein Ende des Rohrkörpers (22), das weggerichtet ist vom Luftbahnkörper (21), den Luftaustritt (13) bildet, und der erste Lufteintrittskanal (12) an einer Position des Luftbahnkörpers (21) vorgesehen ist, die nahe der Innenwand des Rohrkörpers (22) liegt, so dass der blockierende Luftstrom (Q1) zwischen der Innenwand des Rohrkörpers (22) und dem Zerstäubungsgas gebildet wird.

3. Die Struktur (82) zur Bildung eines Luftvorhangs gemäss Anspruch 2, bei der der Luftbahnkörper (21) einen ersten Luftbahnteil (212), einen zweiten Luftbahnteil (213) und einen Wandteil (211) umfasst; und der Luftstromkanal (11) weiter einen Eintrittskanal (111) und einen Luftführungskanal (112) aufweist, die miteinander kommunizieren, wobei der Eintrittskanal (111) im ersten Luftbahnteil (212) vorgesehen ist, der Luftführungskanal (112) im Rohrkörper (22) vorgesehen ist, der zweite Luftbahnteil (213) auf eine Peripherie des Rohrkörpers (22) aufgeschoben ist und der Wandteil (211) mit dem ersten Luftbahnteil (212) und zweiten Luftbahnteil (213) verbunden ist und den Luftführungskanal (112) deckt; und
wobei der erste Lufteintrittskanal (12) am Wandteil (211) vorgesehen ist.

4. Die Struktur (82) zur Bildung eines Luftvorhangs gemäss Anspruch 3, bei der der Luftstromkanal (11) vielzählige erste Lufteintrittskanäle (12) aufweist, die voneinander längs einer Umfangsrichtung des Wandteils (211) beabstandet sind; oder wobei der erste Luftbahnteil (212) weiter einen Lüftungsteil (214) und einen ersten Anschlussteil (215) umfasst, und der Lüftungsteil (214) an den Wandsteil (211) über den ersten Anschlussteil (215) angeschlossen ist; und
ein Querschnittbereich des ersten Anschlussteils (215) kleiner ist als ein Querschnittbereich des Lüftungsteils (214), so dass eine Klemmöffnung (216) an einer Stelle des ersten Anschlussteils (215) gebildet wird, und die Klemmöffnung (216) konfiguriert ist, um eine Vorrichtung zur Erzeugung von Zerstäubungsgas der elektronischen Zerstäubervorrichtung zu klemmen.

5. Die Struktur (82) zur Bildung eines Luftvorhangs gemäss Anspruch 4, bei der ein zweiter Lufteintrittskanal (15) im Lüftungsteil (214) vorgesehen ist, und ein durch den zweiten Lufteintrittskanal (15) eintretender Luftstrom (Q2) fähig ist, das Zerstäubungsgas (G) zwecks Austritt aus dem Luftaustritt (13) zu leiten.

6. Die Struktur (82) zur Bildung eines Luftvorhangs gemäss Anspruch 5, bei der der zweite Lufteintrittskanal (15) einen Lufteintrittsteil (151) und einen Luftleitungsteil (152) aufweist, wobei der Lufteintrittsteil (151) mit dem Luftstromkanal (11) über den Luftleitungsteil (152) kommuniziert, und eine sich ichtung des Luftleitungsteils (152) parallel zu einer Verlängerungsrichtung des Eintrittskanals (111) verläuft; oder
wobei eine umfassende Luftbahn (14) an einem Teil der Klemmöffnung (216) gebildet ist, und die umfassende Luftbahn mit dem ersten Lufteintrittskanal (12) beziehungsweise dem zweiten Lufteintrittskanal (15) kommunisiert; oder
wobei der erste Lufteintrittskanal (12) entsprechend dem zweiten Lufteintrittskanal (15) vorgesehen ist; oder der erste Lufteintrittskanal (12) und der zweite Lufteintrittskanal (15) versetzt sind; oder
wobei ein Querschnittbereich des ersten Lufteintrittskanals (12) grösser ist als ein Querschnittbereich des zweiten Lufteintrittskanals (15); und/oder eine Anzahl der ersten Lufteintrittskanäle (12) grösser ist als eine Anzahl des zweiten Lufteintrittakanals (15).

7. Die Struktur (82) zur Bildung eines Luftvorhangs gemäss Anspruch1, wobei die Struktur zur Bildung eines Luftvorhangs (82) weiter einen zweiten Anschlussteil (32) umfasst und der erste Luftstromführungsteil (31) an die Innenwand des Luftstromkanals (11) über den zweiten Anschlussteil (32 angeschlossen ist; oder wobei vielzählige zweite Anschlussteile (32) zwischen dem ersten Luftstromführungsteil (31) und der Innenwand des Luftstromkanals (11) angeordnet sind, die vielzähligen zweiten Anschlussteile (32) sequentiell längs einer peripherischen Richtung des ersten Luftstromführungsteils (31) beabstandet sind, und wobei der erste Lufteintrittskanal (12) zwischen anliegenden zweiten Anschlussteilen (32) gebildet ist.

8. Die Struktur (82) zur Bildung eines Luftvorhangs gemäss Anspruch 1, bei der ein Winkel zwischen der Stromrichtung des durch den zweiten Lufteintrittskanal (15) eintretenden Luftstroms und einer voreingestellten Richtung zwischen 30° und 45° liegt, die voreingestellte Richtung parallel zu einer Stromrichtung des blockierenden Luftstroms (Q1) verläuft.

9. Die Struktur (82) zur Bildung eines Luftvorhangs gemäss Anspruch 1, wobei die Struktur (82) zur Bildung eines Luftvorhangs weiter einen dritten Anschlussteil (34) aufweist, und der zweite Luftstromführungsteil (33) an den ersten Luftstromführungsteil (31) über den dritten Anschlussteil (34) angeschlossen ist; oder
wobei vielzählige dritte Anschlussteile (34) zwischen dem zweiten Luftstromführungsteil (33) und dem ersten Luftstromführungsteil (31) angeordnet sind, die vielzähligen dritten Anschlussteile (34) sequentiell längs einer peripherischen Richtung des zweiten Luftführungsteils (33) beabstandet sind, und wobei der zweite Lufteintrittskanal (15) zwischen anliegenden dritten Anschlussteilen (34) gebildet ist.

10. Die Struktur (82) zur Bildung eines Luftvorhangs gemäss Anspruch 1, wobei die Struktur (82) zur Bildung eines Luftvorhangs weiter einen zweiten Luftstromführungsteil (33) umfasst, der an einer Seite des ersten Luftführungsteils (31) vom Luftaustritt (13) entfernt angeordnet ist, und der zweite Luftstromführungsteil (33) schräg in einer Richtung, die sich von der Innenwand des Luftstromkanals (11) und vom Luftaustritt (13) entfernt, angeordnet ist, um einen zweiten Lufteintrittskanal (15) zu bilden, und wobei ein Luftstrom, der durch den zweiten Lufteintritskanal (15) einritt, fähig ist, das Zerstäubungsgas (G) zu leiten, um aus dem Luftaustritt (13) ausgegeben zu werden.

11. Die Struktur (82) zur Bildung eines Luftvorhangs gemäss Anspruch 10, wobei die Struktur (82) zur Bildung eines Luftvorhangs ein Zerstäuber für die elektronische Zerstäubungsvorrichtung ist, der Zerstäuber eine Zerstäubungssaugdüse und eine Vorrichtung zur Erzeugung von Zerstäubungsgas umfasst, wobei der zweite Luftstromführungsteil (33) in der Zerstäugungssaugdüse angeordnet ist und der zweite Lufteintrittskanal (15) zwischen dem zweiten Luftstromführungsteil (33) und der Vorrichtung zur Erzeugung von Zerstäubungsgas gebildet ist.

12. Die Struktur (82) zur Bildung eines Luftvorhangs gemäss Anspruch 11, wobei die Struktur (82) zur Bildung eines Luftvorhangs weiter einen konvergierenden Kanal (17) definiert, ein Ende des konvergierenden Kanals (17) ein Lufteintritt ist, das andere Ende des konvergierenden Kanals (17) eine divergierende Öffnung (171) ist und die divergiende Öffnung jeweils mit dem ersten Lufteintrittskanal (12) und dem zweiten Lufteintrittskanal (15) verbunden ist.

13. Die Struktur (82) zur Bildung eines Luftvorhangs gemäss Anspruch 12, wobei eine Mittelachse (α) der divergierenden Öffnung (171) sich durch eine Verbindungsstelle des ersten Luftstromführungsteil (31) und zweiten Luftstromführungsteil (33) erstreckt; oder
wobei eine Mittelachse (α) der divergierenden Öffnung (171) vom Luftaustritt (13) bezüglich einer Verbindung des ersten Luftstromführungsteils (31) und des zweiten Luftstromführungsteils (33) entfernt ist; oder
wobei eine Mittelachse (α) der divergierenden Öffnung (171) nahe des Luftaustritts (13) mit Bezug auf eine Verbindung des ersten Luftstromführungsteils (31) und zweiten Luftstromführungsteils (33) liegt; oder
wobei ein konvergierender Kanal (17) einen ersten Kanalabschnitt (172) und einen zweiten Kanalabschnitt (173) aufweist, die miteinander kommunizieren, eine Endöffnung des ersten Kanalabschnitts (172) entfernt vom zweiten Kanalabschnitt (173) bildet die divergierende Öffnung (171) und eine Endöffnung vom zweiten Kanalabschnitt (173), entfernt vom ersten Kanalabschnitt (172) bildet den Lufteintritt; und eine Verlängerungsrichtung des ersten Kanalabschnitts (172) sich von der des zweiten Kanalabschnitts (173) unterscheidet.

14. Die Struktur (82) zur Bildung eines Luftvorhangs gemäss Anspruch 11, bei der die Vorrichtung zur Erzeugung von Zerstäubungsgas einen Montageteil (60) umfasst, der Montageteil (60) mit einem Montagevorsprung (61) und einer Lüftungsrille (62) versehen ist, der Montagevorsprung (61) konfiguriert ist, um die Zerstäubungssaugdüse zu befestigen, wobei der erste Kanalabschnitt (172) zwischen der Zerstäubungssaugdüse und dem Montageteil (60) ausgebildet ist, und der zweite Kanlabschnitt (173) zwischen der Lüftungsrille (62) und der Zerstäubungssaugdüse gebildet ist; oder
wobei eine Peripherie der Zerstäubungssaugdüse mit Begrenzungsrillen (35) versehen ist, die längs einer entsprechenden Umfangsrichtung gehen, und wobei die Begrenzungsrillen (35) konfiguriert sind, um elastische Ringe aufzunehmen, so dass die Zerstäubungssaugdüse an der Vorrichtung für die Erzeugung von Zerstäubungsgas befestigt ist.

15. Die Struktur (82) zur Bildung eines Luftvorhangs gemäss Anspruch1, wobei die Struktur (82) zur Bildung eines Luftvorhangs ein Zerstäuber für die elektronische Zerstäubungsvorrichtung ist und der Zerstäuber einen Zerstäubungshohlraum (71) definiert; und
wobei der Luftstromkanal (11) im Zerstäubungshohlraum (71) vorgeshen ist, und der erste Lufteintrittskanal (12) an einer Position an einer Unterseite des Zerstäubungshohlraums (71) nahe der Innenwand des Zerstäubungshohlraums vorgesehen ist, so dass der blockierende Luftstrom (Q1) zwischen der Innenwand des Zerstäubungshohlraums (71) und dem Zerstäubungsgas (G) gebildet wird.

16. Die Struktur (82) zur Bildung eines Luftvorhangs gemäss Anspruch 15, bei der der Zerstäuber weiter einen zweiten Lufteintrittskanal (15) definiert, ein Luftstrom, der durch den zweiten Lufteintrittskanal (15) eintritt, die Fähigkeit hat, das Zerstäubungsgas (G) so zu leiten, dass es ab dem Luftaustritt (13) abgeleitet wird, wobei der zweite Lufteintrittskanal (15) an der Unterseite des Zerstäubungshohlraums (71) vorgesehen ist und der erste Lufteintrittskanal (12) nahe einer Kante der Unterseite des Zerstäubungskanals (71) bezüglich des zweiten Lufteintrittskanals (15) liegt.

17. Die Struktur (82) zur Bildung eines Luftvorhangs gemäss Anspruch 16, bei der der erste Lufteintrittskanal (12) jeweils an zwei gegenüberliegenden Seiten des zweiten Lufteintrittskanals (15) vorgesehen ist.

18. Die Struktur (82) zur Bildung eines Luftvorhangs gemäss Anspruch 15, wobei die Struktur (82) zur Bildung eines Luftvorhangs vielzählige erste Lufteintrittskanäle (12) definiert, und die vielzähligen ersten Lufteintrittskanäle (12) untereinander beabstandet sind; und/oder ein Querschnitt des ersten Lufteintrittskanals (12) bandförmig ist.

19. Die Struktur (82) zur Bildung eines Luftvorhangs gemäss Anspruch 1, wobei die Struktur (82) zur Bildung eines Luftvorhangs ein Zerstäuber für die elektronische Zerstäubungsvorrichtung ist, und der Zerstäuber einen Luftaustrittskanal (72) definiert; und
der Luftstromkanal (11) im Luftaustrittskanal (72) vorgesehen ist, und der erste Lufteintrittskanal (12) an einer Seitenwand des Luftaustrittskanals (72) vorgesehen ist, so dass der blockierende Luftstrom (Q1) zwischen der Innenwand des Luftaustrittskanals (72) und dem Zerstäubungsgas (G) gebildet wird.

20. Die Struktur (82) zur Bildung eines Luftvorhangs gemäss Anspruch 19, bei der der Zerstäuber weiter einen Zerstäubungshohlraum (71) definiert, der Zerstäubungshohlraum (71) mit dem Luftaustrittskanal (72) kommuniziert, und der erste Lufteintrittskanal (12) an einem Teil des Luftaustrittskanals (72) nahe des Zerstäubungshohlraums (71) vorgesehen ist; oder
wobei der Zerstäuber vielzählige erste Lufteintrittskanäle (12) definiert, die sequentiell untereinander in einer Umfangsrichtung des Luftaustrittskanals (72) beabstandet sind; oder
wobei der erste Lufteintrittskanal (12) ein Rundloch oder ein Langloch ist.

21. Eine elektronische Zerstäubungsvorrichtung, wobei die elektronische Zerstäubungsvorrichtung einen Hauptkörper (81) und eine Struktur (82) zur Bildung eines Luftvorhangs umfasst, der Hauptkörper (81) an die Structur (82) zur Bildung eines Luftvorhangs angeschlossen ist und wobei die Struktur (82) zur Bildung eines Luftvorhangs wie in jeder der Ansprüche 1-20 beansprucht ist.

## Revendications

1. Structure de formation d'un rideau d'air (82) pour un dispositif électronique d'atomisation, qui comprend :
un canal d'écoulement d'air (11), configuré pour délivrer du gaz d'atomisation (G) ;
un premier canal d'entrée d'air (12), communiqué avec le canal d'écoulement d'air (11) et configuré pour introduire un flux d'air externe dans le canal d'écoulement d'air (11), de sorte qu'un flux d'air de blocage (Q1) est formé entre la paroi intérieure du canal d'écoulement d'air (11) et le gaz d'atomisation (G), et une direction d'écoulement du flux d'air de blocage (Q1) est parallèle à la paroi intérieure du canal d'écoulement d'air (11) ; et
une sortie d'air (13) communiquée avec le canal d'écoulement d'air (11) ;
dans laquelle le premier canal d'entrée d'air (12) est situé près de la paroi intérieure du canal d'écoulement d'air (11), et une sortie du premier canal d'entrée d'air (12) fait face à la sortie d'air (13),
la structure de formation de rideau d'air (82) comprend en outre une première partie de guidage du flux d'air (31), le premier canal d'entrée d'air (12) est formé entre la première partie de guidage du flux d'air (31) et la paroi intérieure du canal d'écoulement d'air (11), et
la première partie de guidage du flux d'air (31) est configurée pour guider le flux d'air introduit par le premier canal d'entrée d'air (12) afin qu'il s'écoule le long de la paroi interne du canal d'écoulement d'air (11), pour former le flux d'air de blocage (Q1),
la structure de formation de rideau d'air (82) comprend en outre une deuxième partie de guidage du flux d'air (33), la deuxième partie de guidage du flux d'air (33) est disposée obliquement dans une direction opposée à la paroi interne du canal d'écoulement d'air (11) et à la sortie d'air (13) pour former un deuxième canal d'entrée d'air (15), et un flux d'air entrant par le deuxième canal d'entrée d'air (15) est utilisé pour guider le gaz d'atomisation (G) à travers la sortie d'air (13) en vue de sa distribution.

2. La structure de formation de rideau d'air (82) selon la revendication 1, dans laquelle la structure de formation de rideau d'air (82) est une buse d'aspiration d'atomisation pour le dispositif électronique d'atomisation ; et
la buse d'aspiration d'atomisation comprend un corps de passage d'air (21) et un corps tubulaire (22), et le canal d'écoulement d'air (11) est prévu dans le corps de passage d'air (21) et le corps tubulaire (22). Une extrémité du corps tubulaire (22) éloignée du corps de passage d'air (21) forme la sortie d'air (13), et le premier canal d'entrée d'air (12) est prévu à une position du corps de passage d'air (21) proche de la paroi intérieure du corps tubulaire (22), de sorte que le flux d'air de blocage (Q1) se forme entre la paroi intérieure du corps tubulaire (22) et le gaz d'atomisation (G).

3. La structure de formation d'un rideau d'air (82) selon la revendication 2, dans laquelle le corps de passage d'air (21) comprend une première portion de passage d'air (212), une seconde portion de passage d'air (213) et une portion de paroi (211) ; et le canal d'écoulement d'air (11) comporte en outre un canal d'entrée (111) et un canal de guidage d'air (112) qui communiquent entre eux, le canal d'entrée (111) étant prévu dans la première portion de passage d'air (212), le canal de guidage d'air (112) étant prévu dans le corps tubulaire (22), la deuxième portion de passage d'air (213) étant manchonnée sur la périphérie du corps tubulaire (22), et la portion de paroi (211) est reliée à la première portion de passage d'air (212) et à la deuxième portion de passage d'air (213) et recouvre le canal de guidage d'air (112) ; et
dans laquelle le premier canal d'entrée d'air (12) est prévu sur la portion de paroi (211).

4. La structure de formation d'un rideau d'air (82) selon la revendication 3, dans laquelle le canal d'écoulement d'air (11) a une pluralité de premiers canaux d'entrée d'air (12) espacés les uns des autres le long d'une direction circonférentielle de la portion de paroi (211) ; ou
dans laquelle la première portion de passage d'air (212) comprend en outre une portion de ventilation (214) et une première portion de connexion (215), et la portion de ventilation (214) est reliée à la portion de paroi (211) par l'intermédiaire de la première portion de connexion (215) ; et
la section transversale de la première portion de connexion (215) est inférieure à la section transversale de la portion de ventilation (214), de sorte qu'une ouverture de serrage (216) est formée à un endroit où la première portion de connexion (215) est formée, et l'ouverture de serrage (216) est configurée pour serrer un dispositif de génération de gaz d'atomisation du dispositif électronique d'atomisation.

5. La structure de formation d'un rideau d'air (82) selon la revendication 4, dans laquelle un second canal d'entrée d'air (15) est prévu dans la portion de ventilation (214), et un flux d'air (Q2) entrant par le second canal d'entrée d'air (15) a la capacité de diriger le gaz d'atomisation (G) pour qu'il sorte à travers la sortie d'air (13).

6. La structure de formation d'un rideau d'air (82) selon la revendication 5, dans laquelle le deuxième canal d'entrée d'air (15) a une portion d'entrée d'air (151) et une portion de guidage d'air (152), où la portion d'entrée d'air (151) est configurée pour introduire le flux d'air externe (Q2) dans la portion de guidage d'air (152), la portion d'entrée d'air (151) est communiquée avec le canal d'écoulement d'air (11) à travers la portion de guidage d'air (152), et une direction de prolongement de la portion de guidage d'air (152) est parallèle à une direction de prolongement du canal d'entrée (111) ; ou
dans laquelle un passage d'air complet (14) est formé à une position de l'ouverture de serrage (216), et le passage d'air complet (14) est respectivement en communication avec le premier canal d'entrée d'air (12) et le deuxième canal d'entrée d'air (15) ; ou
dans laquelle le premier canal d'entrée d'air (12) est prévu pour correspondre au deuxième canal d'entrée d'air (15) ; ou le premier canal d'entrée d'air (12) et le deuxième canal d'entrée d'air (15) sont décalés ; ou
dans laquelle la section transversale du premier canal d'entrée d'air (12) est supérieure à la section transversale du deuxième canal d'entrée d'air (15) ; et/ou une quantité de premiers canaux d'entrée d'air (12) est supérieure à une quantité de deuxièmes canaux d'entrée d'air (15).

7. La structure de formation d'un rideau d'air (82) selon la revendication 1, dans laquelle la structure de formation d'un rideau d'air (82) comprend en outre une deuxième portion de connexion (32), et la première portion de guidage du flux d'air (31) est reliée à la paroi intérieure du canal d'écoulement d'air (11) par l'intermédiaire de la deuxième portion de connexion (32) ; ou
dans laquelle une pluralité de deuxièmes portions de connexion (32) sont disposées entre la première portion de guidage du flux d'air (31) et la paroi interne du canal d'écoulement d'air (11), où la pluralité de deuxièmes portions de connexion (32) sont séquentiellement espacées le long d'une direction circonférentielle de la première portion de guidage du flux d'air (31), et où le premier canal d'entrée d'air (12) est formé entre les deuxièmes portions de connexion adjacentes (32).

8. La structure de formation d'un rideau d'air (82) selon la revendication 1, dans laquelle un angle entre une direction d'écoulement du flux d'air entrant par le second canal d'entrée d'air (15) et une direction prédéfinie est compris entre 30° et 45°, et la direction prédéfinie est parallèle à une direction d'écoulement du flux d'air de blocage (Q1).

9. La structure de formation d'un rideau d'air (82) selon la revendication 1, dans laquelle la structure de formation d'un rideau d'air (82) comprend en outre une troisième portion de connexion (34), et la deuxième portion de guidage du flux d'air (33) est reliée à la première portion de guidage du flux d'air (31) par le biais de la troisième portion de connexion (34) ; ou
dans laquelle une pluralité de troisièmes portions de connexion (34) sont disposées entre la deuxième portion de guidage du flux d'air (33) et la première portion de guidage du flux d'air (31). La pluralité des troisièmes portions de connexion (34) sont séquentiellement espacées le long d'une direction circonférentielle de la deuxième portion de guidage du flux d'air (33), et le deuxième canal d'entrée d'air (15) est formé entre les troisièmes portions de connexion adjacentes (34).

10. La structure de formation d'un rideau d'air (82) selon la revendication 1, dans laquelle la structure de formation d'un rideau d'air (82) comprend en outre une deuxième portion de guidage du flux d'air (33) disposée sur un côté de la première portion de guidage du flux d'air (31) à l'écart de la sortie d'air (13), et la deuxième portion de guidage du flux d'air (33) est disposée obliquement dans une direction opposée à la paroi intérieure du canal d'écoulement d'air (11) et de la sortie d'air (13) pour former un deuxième canal d'entrée d'air (15), et un flux d'air entrant par le deuxième canal d'entrée d'air (15) est capable de guider le gaz d'atomisation (G) pour qu'il soit émis à travers la sortie d'air (13).

11. La structure de formation d'un rideau d'air (82) selon la revendication 10, dans laquelle la structure de formation d'un rideau d'air (82) est un atomiseur pour le dispositif d'atomisation électronique, où l'atomiseur comprend une buse d'aspiration d'atomisation et un dispositif de génération de gaz d'atomisation, où la deuxième portion de guidage du flux d'air (33) est disposée dans la buse d'aspiration d'atomisation, et où le deuxième canal d'entrée d'air (15) est formé entre la deuxième portion de guidage du flux d'air (33) et le dispositif de génération de gaz d'atomisation.

12. La structure de formation d'un rideau d'air (82) selon la revendication 11, dans laquelle la structure de formation d'un rideau d'air (82) définit en outre un canal convergent (17), où l'une des extrémités du canal convergent (17) est une entrée d'air, l'autre extrémité du canal convergent (17) est une ouverture divergente (171), et l'ouverture divergente (171) est communiquée respectivement avec le premier canal d'entrée d'air (12) et le deuxième canal d'entrée d'air (15).

13. La structure de formation d'un rideau d'air (82) selon la revendication 12, dans laquelle une ligne centrale (α) de l'ouverture divergente (171) s'étend à travers un joint de la première portion de guidage du flux d'air (31) et de la seconde portion de guidage du flux d'air (33) ; ou
dans laquelle une ligne centrale (α) de l'ouverture divergente (171) est éloignée de la sortie d'air (13) par rapport à un joint de la première partie de guidage du flux d'air (31) et de la deuxième partie de guidage du flux d'air (33) ; ou
dans laquelle une ligne centrale (α) de l'ouverture divergente (171) est proche de la sortie d'air (13) par rapport à un joint de la première partie de guidage du flux d'air (31) et de la deuxième partie de guidage du flux d'air (33) ; ou
dans laquelle le canal convergent (17) a une première section de canal (172) et une deuxième section de canal (173) qui sont en communication l'une avec l'autre, une ouverture d'extrémité de la première section de canal (172) éloignée de la deuxième section de canal (173) est l'ouverture divergente (171) et une ouverture d'extrémité de la deuxième section de canal (173) éloignée de la première section de canal (172) est l'entrée d'air ; et une direction d'extension de la première section de canal (172) est différente de celle correspondant à la deuxième section de canal (173).

14. La structure de formation d'un rideau d'air (82) selon la revendication 11, dans laquelle le dispositif de génération de gaz d'atomisation comprend une portion de montage (60), la portion de montage (60) étant pourvue d'une saillie de montage (61) et d'une rainure de ventilation (62). La saillie de montage (61) est configurée pour fixer la buse d'aspiration d'atomisation, la première section de canal (172) est formée entre la buse d'aspiration d'atomisation et la portion de montage (60), et la deuxième section de canal (173) est formée entre la rainure de ventilation (62) et la buse d'aspiration d'atomisation ; ou
dans laquelle la périphérie de la buse d'aspiration d'atomisation est pourvue de rainures de limitation (35) s'étendant le long d'une direction circonférentielle respective, et les rainures de limitation (35) sont configurées pour recevoir des anneaux élastiques, de sorte que la buse d'aspiration d'atomisation est fixée au dispositif de génération de gaz d'atomisation.

15. La structure de formation d'un rideau d'air (82) selon la revendication 1, dans laquelle la structure de formation d'un rideau d'air (82) est un atomiseur pour le dispositif électronique d'atomisation, et l'atomiseur définit une cavité d'atomisation (71) ; et
dans laquelle le canal d'écoulement d'air (11) est prévu dans la cavité d'atomisation (71), et le premier canal d'entrée d'air (12) est prévu à une position sur le fond de la cavité d'atomisation (71) près de la paroi intérieure de la cavité d'atomisation (71), de sorte que le flux d'air de blocage (Q1) est formé entre la paroi intérieure de la cavité d'atomisation (71) et le gaz d'atomisation (G).

16. La structure de formation d'un rideau d'air (82) selon la revendication 15, dans laquelle l'atomiseur définit en outre un deuxième canal d'entrée d'air (15), un flux d'air entrant par le deuxième canal d'entrée d'air (15) étant capable de guider le gaz d'atomisation (G) pour qu'il puisse être évacué par la sortie d'air (13), le second canal d'entrée d'air (15) étant prévu sur le fond de la cavité d'atomisation (71), et le premier canal d'entrée d'air (12) étant situé proche d'un bord du fond de la cavité d'atomisation (71) par rapport au deuxième canal d'entrée d'air (15).

17. La structure de formation d'un rideau d'air (82) selon la revendication 16, dans laquelle le premier canal d'entrée d'air (12) est respectivement prévu sur deux côtés opposés du second canal d'entrée d'air (15).

18. La structure de formation d'un rideau d'air (82) selon la revendication 15, dans laquelle la structure de formation d'un rideau d'air (82) définit une pluralité de premiers canaux d'entrée d'air (12), et la pluralité de premiers canaux d'entrée d'air (12) sont espacés les uns des autres ; et/ou la section transversale du premier canal d'entrée d'air (12) a la forme d'une bande.

19. La structure de formation d'un rideau d'air (82) selon la revendication 1, dans laquelle
la structure de formation d'un rideau d'air (82) est un atomiseur pour le dispositif électronique d'atomisation, et l'atomiseur définit un canal de sortie d'air (72) ; et
le canal d'écoulement d'air (11) est prévu dans le canal de sortie d'air (72) et le premier canal d'entrée d'air (12) est prévu sur une paroi latérale du canal de sortie d'air (72), de sorte que le flux d'air de blocage (Q1) est formé entre la paroi intérieure du canal de sortie d'air (72) et le gaz d'atomisation (G).

20. La structure de formation d'un rideau d'air (82) selon la revendication 19, dans laquelle l'atomiseur définit en outre une cavité d'atomisation (71), la cavité d'atomisation (71) est en communication avec le canal de sortie d'air (72) et le premier canal d'entrée d'air (12) est prévu sur une portion du canal de sortie d'air (72) située à proximité de la cavité d'atomisation (71) ; ou
dans laquelle l'atomiseur définit une pluralité de premiers canaux d'entrée d'air (12) séquentiellement espacés les uns des autres dans une direction circonférentielle du canal de sortie d'air (72) ; ou
dans laquelle le premier canal d'entrée d'air (12) est un trou circulaire ou un trou oblong.

21. Un dispositif électronique d'atomisation, dans lequel le dispositif électronique d'atomisation comprend un corps principal (81) et une structure de formation d'un rideau d'air (82), où le corps principal (81) est connecté à la structure de formation d'un rideau d'air (82), et la structure de formation d'un rideau d'air (82) est telle que revendiquée dans une quelconque des revendications 1 à 20.
